# FASCICULE DE BREVET EUROPEEN

(11) **EP 0 922 700 B1**
(45) Date de publication et mention de la délivrance du brevet: **11.09.2002**
(21) Numéro de dépôt: 98403108.8
(22) Date de dépôt: 09.12.1998
(51) Int. Cl.: C07D 257/02, A61K 49/00

(54) **Chelates métalliques de dérivés macrocycliques polyaminocarboxyliques et leur application à l'imagerie diagnostique**
Metallchelate von makrocyclischen polyaminocarbocyclischen Derivaten und deren Verwendung zur Diagnostik-Bilderzeugung
Metal chelates of macrocyclic polyaminocarboxylic derivatives and their use for the diagnostic imaging

(30) Priorité: 10.12.1997 FR 9715642
(43) Date de publication de la demande: 16.06.1999
(73) Titulaire: GUERBET, 93420 Villepinte (FR)
(72) Inventeur: Meyer, Dominique, 94100 Saint-Maur (FR); Port, Marc, 95170 Deuil-la-Barre (FR); Rousseaux, Olivier, 60300 Senlis (FR); Simonot, Christian, 75020 Paris (FR)
(74) Mandataire: Bernasconi, Jean Raymond

(56) Documents cités:
- EP-A- 0 661 279
- FR-A- 2 736 051

## Description

La présente invention concerne des chelates formés par des acides polyaminocarboxyliques macrocycliques avec des cations métalliques et leur utilisation en imagerie diagnostique, notamment par résonance magnétique (IRM) pour des cations magnétiques.

Les relaxivités r₁ et r₂ et particulièrement la première donnent la mesure de l'efficacité magnétique d'un tel chelate et permettent d'apprécier son utilité comme produit de contraste. Pour les produits commercialisés actuellement, r₁ ne dépasse pas 6 mM⁻¹s⁻¹ (à 20 MHz et 37°C), que les chelates soient linéaires (gadopentetate ou DTPA et gadoversetamide) ou macrocycliques (gadoterate ou DOTA et gadoteridol). En outre, ces composés, administrés par voie intraveineuse, diffusent rapidement hors du domaine vasculaire vers le compartiment interstitiel extracellulaire et il n'y a pas encore sur le marché de produit qui reste suffisamment localisé dans les vaisseaux pour permettre une bonne évaluation de la perfusion tissulaire, de la perméabilité capillaire ou du volume sanguin.

Des dérivés macromoléculaires résultant du greffage d'un chelate classique sur un polymère biocompatible, comme celui obtenu par fixation du gadopentetate sur le polyethylène glycol ou la polylysine, ou du gadopentetate et du gadoterate sur un polysaccharide, décrits depuis plus de 10 ans et qui ont été testés chez l'animal, ne sont toujours pas développés chez l'homme.

On a depuis proposé le greffage de ces chelates classiques (DTPA ou DOTA) sur des polymères ramifiés de masse moléculaire unique, dits en cascade ou dendrimères, constitués d'unités répétitives variées, comme par exemple dans EP-A-430863 ou EP-A-607222. Il est mentionné que les relaxivités de ces produits sont élevées et qu'ils présentent une certaine rémanence vasculaire mais ces résultats restent limités puisque, par exemple, les polyamines portant 24 ou 48 molécules de gadopentetate décrites dans EP-A-430863 n'ont, malgré leur taille, qu'une relaxivité r₁ de l'ordre de 13 mM⁻¹s⁻¹, pour 4,8 mM⁻¹s⁻¹ pour le gadopentetate seul; en outre, il paraît difficile d'isoler lors de la synthèse un composé unique, à savoir d'obtenir la substitution de tous les groupes terminaux d'une telle polyamine, en supposant qu'on ait pu l'obtenir de masse moléculaire uniforme, ce qui éviterait la dispersité des propriétés physicochimiques et pharmacocinétiques des composants de la dose administrée, inconvénient connu des dérivés de polymères classiques, qui a limité leur développement.

Dans les travaux les plus récents visant à l'obtention de molécules à forte relaxivité, de masse moléculaire unique et ne comportant qu'un seul groupement chelateur, on peut citer EP-A-661279 et WO 97/01359 qui soulignent l'intérêt de l'introduction, dans les structures moléculaires connues, d'au moins 3 bras latéraux hydrophiles de masse supérieure à 200 sur les substituants latéraux des atomes d'azote donneurs qui portent les autres groupes de coordination, qu'ils soient des groupes acides ou dérivés; notamment, il est indiqué que les complexes de gadolinium des composés dérivés du gadoterate de formule 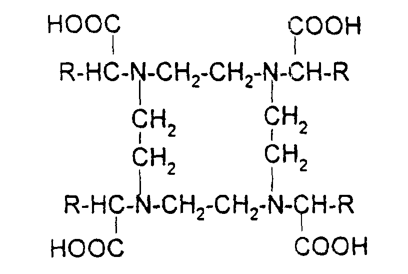 dans laquelle 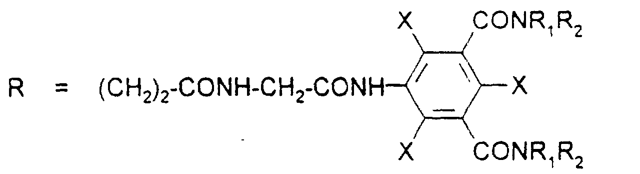 ont une relaxivité r₁>20 mM⁻¹s⁻¹ à 20 MHz et 37°C.

Comme composés à forte relaxivité et présentant une certaine rémanence vasculaire, il faut aussi citer ceux décrits dans WO 96/23526, de structure sensiblement différente des précédents, dont l'un des représentants, MS325, complexe de gadolinium du composé de formule 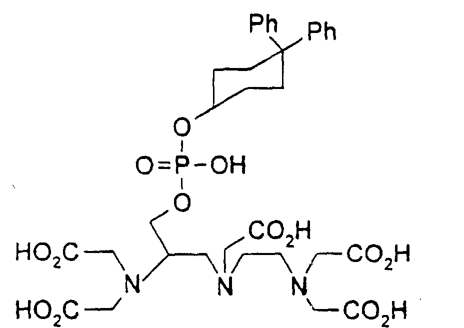 se fixe réversiblement sur l'albumine du plasma de telle sorte que sa relaxivité r₁ dans ce milieu est pratiquement 10 fois celle du gadopentetate dont il dérive, pour une demi-vie multipliée par 4 et un volume de distribution divisé par 2,5 chez le lapin d'après Acad. Radiol. S356-S358 (1996).

Contrairement au MS325 et ses homologues, les complexes métalliques paramagnétiques de la présente invention ont des relaxivités élevées, même dans l'eau, puisque r₁ y est supérieure à 30 mM⁻¹s⁻¹ (20 MHz, 37° C); en outre, alors qu'ils ne se fixent pratiquement pas sur l'albumine plasmatique, ils présentent une nette localisation vasculaire et sont principalement éliminés par les reins de telle sorte qu'ils peuvent être avantageusement utilisés en IRM comme produits de contraste, révélateurs du système circulatoire.

Selon un premier aspect, l'invention concerne les chelates d'ions métalliques paramagnétiques, ou radioactifs, des macrocycles polyaminocarboxyliques de formule 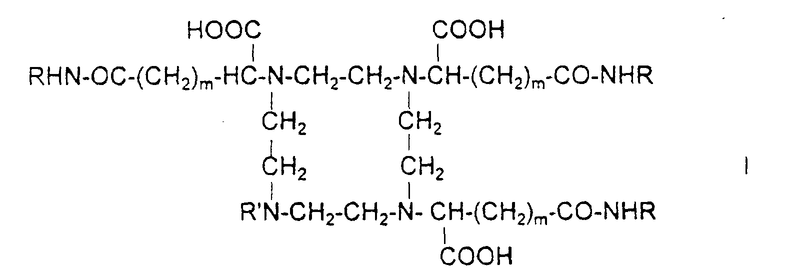 dans laquelle
m est 1 ou 2,
R' est H, un groupe (C₁-C₄) alkyl, éventuellement hydroxylé, un groupe CH₂-COOH, CH₂-CONZ₁Z₂, Z₁ et Z₂ étant indépendamment l'un de l'autre, H, ou un groupe (C₁-C4) alkyl, éventuellement hydroxylé,
ou R' est un groupe HOOC-CH-(CH₂)ₘ-CONHR,
R est un groupe 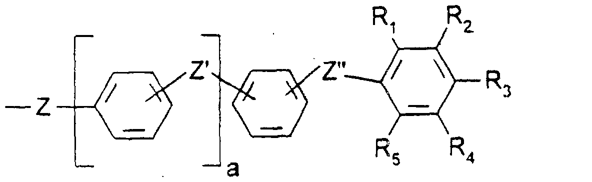 dans lequel a est 1 ou 2
Z est une liaison, CH₂, CH₂-CO-NH ou (CH₂)₂-NHCO
Z' est une liaison, O, S, NQ, CH₂, CO, CO-NQ, NQ-CO, NQ-CO-NQ, CO-NQ-CH₂-CONQ
Z" est CO-NQ, NQ-CO ou CO-NQ-CH₂-CO-NQ, NQ-CO-CH₂-NQ-CO avec Q est H ou un groupe (C₁-C₄) alkyl, éventuellement hydroxylé
R₁, R₂, R₃, R₄, R₅, indépendamment l'un de l'autre, sont choisis parmi H, Br, Cl, I, CO-NQ₁Q₂ ou N(Q₁)-CO-Q₂ et Q₁ et Q₂, identiques ou différents, sont choisis parmi les groupes (C₂-C₆) alkyl éventuellement hydroxylés et éventuellement interrompus par l'atome d'oxygène, de telle sorte que Q₁ et Q₂ comportent à eux deux de 4 à 10 groupes OH,
étant entendu qu'au moins 1, et au plus 2, groupes R₁, R₂, R₃, R₄, R₅ sont des groupes amides.

Les cations métalliques complexés dans les chelates de l'invention sont choisis parmi les cations paramagnétiques, généralement utilisés en IRM, spécialement les cations trivalents dont Fe³⁺, Cr³⁺, Eu³⁺, Gd³⁺, Tb³⁺, et particulièrement Gd³⁺, ou pour une utilisation en scintigraphie parmi les cations des radioéléments tels que ^{99m}Tc ou ¹¹¹In.

Les bases minérales qui peuvent donner un sel physiologiquement acceptable avec l'éventuelle fonction acide libre d'un complexe métallique du composé de formule I, sont préférentiellement choisies parmi les hydroxydes et les carbonates des métaux alcalins (K⁺, Na⁺) ou alcalino terreux (Ca⁺⁺, Mg⁺⁺), tandis que les bases organiques sont choisies parmi les amines telles qu'aminoéthanol, throméthamine ou N-méthylglucamine, ou parmi les amino-acides tels que la lysine, la glycine ou l'arginine.

On préfère les composés dans lesquels m est 2 et a est 1, R' n'est pas H ou alkyl, Z est une liaison, CH₂ ou CH₂CONH, Z' est CONH, NHCO, CONHCH₂CONH ou NHCONH,
Z" est CONH ou CONHCH₂CONH avec R₃ = CONQ₁Q₂ et R₁, R₂, R₄, R₅ sont indépendamment H, Br, Cl ou I, ou R₂ = R₄ = CONQ₁Q₂, auquel cas R₁, R₃, R₅ sont indépendamment H, Br, Cl ou I,
ou Z" est NHCO avec R₃ = N(Q₁)COQ₂ et R₁, R₂, R₄, R₅ sont indépendamment H, Br, Cl ou I, ou R₂ = R₄ = N(Q₁)COQ₂, auquel cas R₁, R₃, R₅ sont indépendamment H, Br, Cl ou I.

Parmi ceux-ci on préfère les composés dans lesquels Z' est CONH, CONHCH₂CONH ou NHCONH, et mieux, ceux pour lesquels Z' est CONH, liaison plus courte, R₁ R₃, R₅ sont tous les trois Br ou I et R₂ = R₄ = CONQ₁Q₂ avec Q₁ et Q₂ sont des groupes (C₂-C₆) alkyl hydroxylés comportant à eux deux 6 à 10 groupes OH ou si Q₁ et/ou Q₂ est interrompu par un atome d'oxygène, de 4 à 8 groupes OH.

Les chelates métalliques des composés de formule I peuvent être préparés par un procédé qui consiste à faire réagir sur un chelate II, du cation métallique M³⁺ et du composé de formule 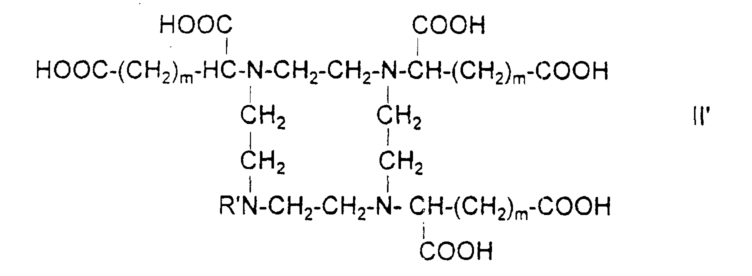 une amine de formule 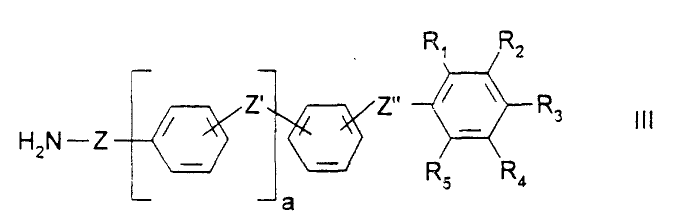 dans laquelle les lettres ont la même signification que dans la formule I en présence d'un agent de couplage, notamment ceux utilisés en synthèse peptidique, adapté au milieu réactionnel, aqueux ou organique, dans lequel les composés de formule II et III sont en solution, au moins partiellement. Dans ces conditions, les groupes acides carboxyliques bloqués situés sur les carbones en alpha des atomes d'azote du composé II ne réagissent pas.

Selon un autre aspect, l'invention concerne les produits de contraste pour imagerie chez l'homme ou l'animal par scintigraphie lorsque le chelate est radiomarqué ou par résonance magnétique lorsque le chelate comporte un ion paramagnétique, éventuellement sous forme de sel d'un cation minéral ou organique, dans un véhicule pharmaceutiquement acceptable, associé à des excipients compatibles.

Les composés de l'invention peuvent être utilisés dans des méthodes d'imagerie du corps humain ou animal qui consistent à administrer à un sujet une dose unitaire efficace d'une composition de l'invention et à soumettre le tissu ou la zone à étudier à un champ magnétique convenable pour observer la résonance magnétique du proton, ou à une scintigraphie lorsque le chelate contient un radioélément.

Les chelates des composés de formule I qui comportent 4 substituants identiques sur les atomes d'azote du macrocycle central, le 1,4,7,10-tétraazacyclododécane, ou cyclen, sont préparés avec un nombre d'étapes inférieur à celui des cas dans lesquels R' représente H, CH₂COOH, CH₂CONZ₁Z₂, ou un groupe alkyl ou hydroxyalkyl en C₁-C₄, tel que CH₃, CH₂CH₂OH ou CH₂-CHOH-CH₂OH, puisqu'on doit bloquer temporairement sélectivement l'un des 4 atomes d'azote du cyclen par un groupe labile ou effectuer préalablement une monosubstitution du macrocycle par le groupe R'.

Les groupes R des composés de l'invention comportent 3 ou 4 noyaux phényles, dont celui le plus éloigné du macrocycle est substitué par un ou des groupes hydrophiles, qui influent sur la solubilité aqueuse et la biocompatibilité du chelate. Parmi ces groupes hydrophiles, on préfère les groupes amides tertiaires, dont les substituants sont des groupes (C₂-C₆) alkyl hydroxylés éventuellement interrompus par un atome d'oxygène et particulièrement ceux dérivés des aminoalcools linéaires HNQ₁Q₂ dans lesquels Q₁ et Q₂, indépendamment l'un de l'autre, sont CH₂(CHOH)ₙ(CH₂OCH₂)ᵣ(CHOH)ₚCH₂OH, et,
si dans Q₁ et Q₂ r = 0, alors p = 0 et n peut être 0 à 4 et Q₁ et Q₂ comportent ensemble de 5 à 10 groupes OH, ce qui correspond notamment pour HNQ₁Q₂ à H-N[CH₂(CHOH)₄CH₂OH]₂ et 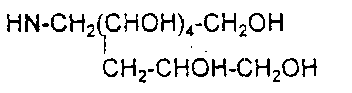 si r = 1 dans Q₁ et/ou Q₂, n et p sont, indépendamment l'un de l'autre, 0 ou 1 et Q₁ et Q₂ comportent ensemble de 4 à 8 groupes OH, ce qui correspond notamment pour HNQ₁Q₂ à 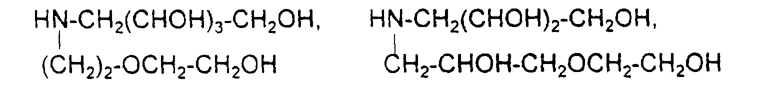 et

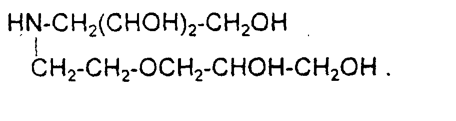

Ces aminoalcools précurseurs sont commerciaux ou peuvent être préparés
- soit à partir de l'aminoalcool primaire convenable, par exemple par action d'un sucre et réduction comme décrit dans EP-A-675105 lorsque n = 4 et p = r = 0, ou pour ceux dans lesquels n = 3 et p = r = 0, comme décrit dans EP-A-558395,
- soit par action d'un sucre sur la benzylamine suivie de l'introduction du second substituant hydroxylé par action d'un halogénure ou sulfonate convenable puis de l'élimination du benzyle par hydrogénation catalytique.

Lorsque Q₁ et/ou Q₂ comportent un atome d'oxygène dans la chaîne, les aminoalcools sont, lorsque n = p = 0, préparés à partir du 2-aminoéthoxyéthanol sur lequel on peut faire réagir un époxyde ou un halogénure d'alkyl hydroxylé convenable ou encore un aldéhyde aliphatique hydroxylé tel qu'un monosaccharide pour former une imine, ensuite réduite par voie catalytique ou chimique.

Lorsque n est 1 on peut préparer les aminoalcools par action de l'époxyde 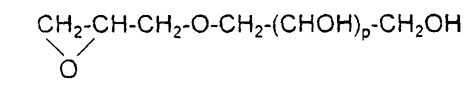 sur l'aminoalcool primaire convenable, les dits époxydes étant obtenus par oxydation des dérivés éthyléniques correspondants, par un peracide ou un acide peroxyimidique, comme décrit dans J. Org. Chem. 26 659-663 (1961) et 48 888-890 (1983).

La présence d'atomes d'halogène à côté des groupes amides sur le noyau phényle est avantageuse et on préfère les groupes R dans lesquels R₁=R₃=R₅=Br ou I et R₂=R₄=CO-NQ₁Q₂, notamment pour leur stabilité.

On peut former le pont Z" entre les 2 noyaux phényles avant ou après les ponts Z'.

Par exemple, le composé 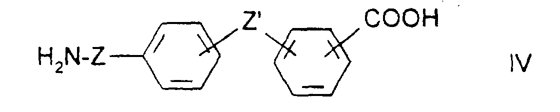 peut être préparé, quand Z est une liaison, à partir des dérivés diphényles V ou leurs esters 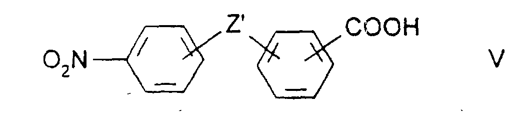 dans lesquels Z' a la même signification que dans la formule I.

Le composé V dans lequel Z' est 0 est décrit dans Makromoleculare Chemie 130 103-144 (1969); celui dans lequel Z' est NH est décrit dans Indian J. Chem. 13 35-37 (1975), celui dans lequel Z' est CH₂ ou CO, dans J. Pharm. Sci. 55(3) 295-302 (1966), celui dans lequel Z' est une liaison, dans Synth. Comm. 24(22) 3307-3313 (1994), et celui dans lequel Z' est S est décrit dans II Farmaco, 44(7-8) 683-684 (1989).

D'autres composés V peuvent être préparés par des procédés d'analogie; par exemple, lorsque Z' est HNCONH, par action de O₂NC₆H₄NCO sur H₂NC₆H₄COOH en milieu anhydre, ou lorsque Z' est NHCO ou CONH, par réaction du chlorure d'acide aromatique sur l'aniline convenable en solution dans un solvant aprotique tel que CH₂Cl₂, C₆H₅CH₃, CH₃CON(CH₃)₂, ou de l'acide aromatique sur l'aniline en présence d'un chlorure d'acide sulfonique, de triéthylamine et de diméthylaminopyridine comme décrit dans Synth. Communications 25(18) 2877-2881 (1995).

La réduction du groupe NO₂ en NH₂ peut être effectuée, de manière connue, par l'hydrogène en présence de catalyseur, ou par voie chimique.

Lorsque Z dans la formule IV est CH₂-CONH, on fait réagir la glycine activée dans laquelle le groupe NH₂ est protégé, sur le composé IV dans lequel Z est une liaison ou sur une aniline portant un groupe précurseur de Z', éventuellement protégé. La glycine est protégée par exemple sous forme de carbamate, en particulier t-butylcarbamate (Synthesis, 48, 1986) et benzylcarbamate (Chem., Ber., 65, 1192 (1932)), sous forme de phtalimide (Tetrahedron Letters 25, 20, 2093-2096 (1984)), avec un benzyl (Bull. Soc. Chim. Fr., 1012-1015, (1954)), un N-allyl (Tetrahedron Letters 22, 16, 1483-1486 (1981)). (Voir aussi Protective groups in organic synthesis 315-349, T.W. Greene (John Wiley & Sons Inc.).)

L'élimination du groupe protecteur du NH₂ fixé sur Z n'est, en général, effectuée qu'au stade du composé III. De façon classique, un groupe phtalimido est éliminé par action de l'hydrazine, alors qu'un groupe benzyloxycarbonyle ou benzyle l'est par hydrogénation catalytique.

Lorsque Z=CH₂ dans le composé IV et Z'=CONH ou CONHCH₂CONH, on peut faire réagir l'acide 4-aminométhylbenzoïque dans lequel le groupe NH₂ est protégé sous forme de carbamate ou d'imide comma décrit dans J. Org. Chem. 43, 2320-2325 (1978) ou dans Rec. Trav. Chim. Pays-Bas, 79, 688 (1960) sur l'acide benzoïque protégé convenablement substitué.

Lorsque Z est (CH₂)₂NHCO, on peut préparer le composé III par action d'un excès d'éthylènediamine sur un ester benzoïque convenable, soit portant l'enchaînement (Z'-phényle)ₐ-Z"-phényle substitué, soit portant seulement un groupe précurseur de Z' éventuellement protégé.

Par ailleurs, lorsque a est égal à 2, on peut faire réagir le composé IV dont le groupe amine est éventuellement protégé sur un acide aminobenzoïque dont la fonction acide est éventuellement protégée, pour obtenir les composés IV' dans lesquels le second Z' est CONH de formule

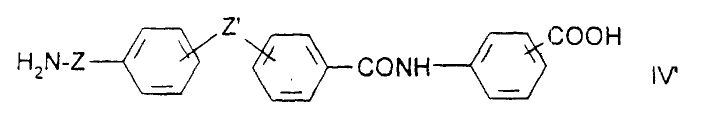

On fait ensuite réagir sur le noyau phényle terminal convenablement substitué de formule 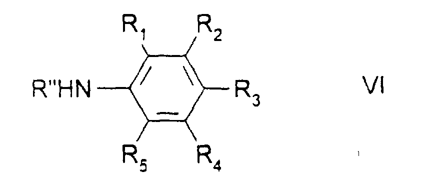 dans lequel R₁, R₂, R₃, R₄, R₅ sont H, halogène, CO-NQ₁Q₂ comme dans la formule I ou sont éventuellement protégés et R" représente CO-CH₂-NH₂ ou H, en solution organique ou aqueuse, les composés IV ou IV' soit sons forme de chlorure d'acide soit en présence d'un agent de couplage, tel que ceux utilisés en synthèse peptidique et par exemple un chloroformiate, un sulfochlorure, ou un carbodiimide aliphatique, éventuellement porteur d'un groupe amine ou ammonium quaternaire solubilisant.

L'amide de formule VI dans lequel R" = H est préparé par des méthodes connues de l'homme du métier à partir de l'aminoacide aromatique convenable, éventuellement halogéné, dans lequel chaque groupe carboxylique présent est activé sous forme de chlorure d'acide ou d'anhydride mixte, ou à partir de l'acide aromatique nitré que l'on amidifie avant de réduire le groupe NO₂.

Les composés VI dans lequels R₁=R₃=R₅= Br ou I, et R₂ et R₄ = CO-NQ₁Q₂ avec Q₁ = CH₂(CHOH)₄CH₂OH et Q₂ = CH₂CH₂OH ou CH₂(CHOH)₁₋₃-CH₂OH et R" =CO-CH₂-NH₂ sont décrits dans WO 97/01359. Les autres dérivés, halogénés ou non, et notamment ceux dans lesquels les chaînes Q₁ et Q₂ sont interrompues par un atome d'oxygène peuvent être préparés de manière analogue à partir des aminoalcools convenables.

Lorsque Z est une liaison on préfère faire réagir le composé nitré tel que V sur l'aniline VI avant d'effectuer la réduction.

On peut aussi éventuellement, faire réagir les composés IV ou IV' sur un composé précurseur de VI, comportant le groupe COOH, éventuellement protégé sous forme d'ester, à la place du groupe CO-NQ₁Q₂, pour ne faire qu'ensuite le mono ou diamide, ou encore faire réagir un composé 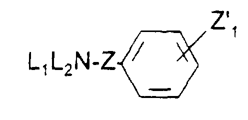 dans lequel NL₁L₂ est un groupe amino protégé, avec 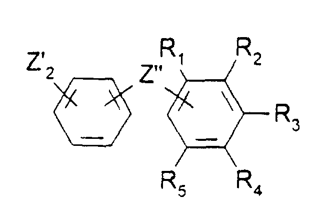 tels que Z'₁ et Z'₂ réagissent pour donner Z'.

Pour préparer un composé III dans lequel R₃ ou R₂ et R₄ représentent N(Q₁)CO-Q₂ et Z" = NH-CO ou NH-CO-CH₂-NH-CO, on peut faire réagir l'acide benzoïque de formule 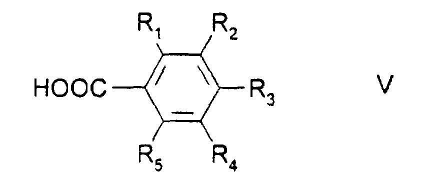 dans lequel les hydroxyles du groupe N(Q₁)COQ₂ sont éventuellement protégés sur une amine 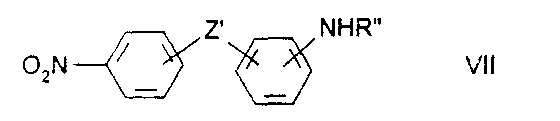 dans laquelle R" est H ou COCH₂-NH₂, avant de réduire le groupe nitro pour obtenir un composé III dans lequel Z est une liaison.

Parmi les composés de formule V, on peut citer ceux préparés à partir de CH₂(OCOCH₃)CH(OCOCH₃)₄COCl, décrit dans Org. Synth. 41, 79-82 (1961), et des acides aminobenzoïques convenables, ou celui dans lequel R₁= R₃= R₅ = I et R₂= R₄= 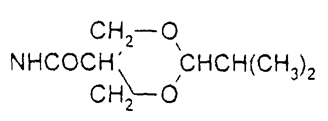 décrit dans EP 357467.

Dans les groupes amides Z' et Z", Q peut être introduit de façon classique par action de l'halogénure correspondant en présence d'une base sur le composé dans lequel Q = H, de préférence aux stades des intermédiaires.

La réaction des composés de formule III sur les sels des chelates de formule II a lieu, de préférence en milieu aqueux, éventuellement en présence d'un tiers solvant aprotique polaire tel que dioxanne ou tétrahydrofurane, en présence d'un carbodiimide soluble, tel que ceux porteurs du groupe amine décrits dans J. Org. Chem. 21, 439-441 (1956) et 26, 2525-2528, (1961) ou US 3,135,748, ou de groupe ammonium quaternaire dans Org. Synth. V, 555-558, qui concerne un dérivé du 1-éthyl-3-(3-diméthylamino)propyl carbodiimide (EDCI) ou son analogue, le 1-cyclohexyl 3-(2-morpholinoéthyl)carbodiimide métho p-tolyl sulfonate. Elle peut aussi être effectuée en présence de N-hydroxy-sulfosuccinimide comme décrit dans Bioconjugate Chem. 5, 565-576 (1994) ou de 2-succinimido 1,1,3,3-tétraméthyluronium tétrafluoroborate et analogues décrits dans Tetrahedron Letters 30, 1927-1930 (1989).

Un autre procédé consiste à former un ester activé intermédiaire en faisant réagir par exemple le N-hydroxysulfosuccinimide (NHS) ou l'hydroxybenzotriazole (HOBT) en présence de carbodiimide tel que l'EDCI sur le chelate II qui peut être solubilisé par salification avec un cation minéral, par exemple un ammonium ou sodium.

En présence de 2-éthoxy 1-éthoxycarbonyl 1,2-dihydroquinoléine (EEDQ), on peut effectuer la réaction en milieu hydroalcoolique.

Le composé de formule II' dans laquelle m est 1, peut être préparé à partir du cyclen sur lequel on fait réagir un diester de l'acide acétylène dicarboxylique pour obtenir une énamine que l'on réduit par une méthode classique, catalytique ou chimique, suivie de l'hydrolyse des fonctions esters. Dans le cas d'ester benzylique, on préfère dans une première étape, effectuer la réduction chimique des énamines, par exemple par action d'un cyanoborohydrure, suivie de la débenzylation par l'hydrogène en présence de catalyseur.

Le chelate II dans lequel m = 2 et les 4 substituants des atomes d'azote sont identiques et M³⁺ est Gd³⁺ est un composé connu, décrit notamment dans EP-A-661279.

Le chelate II dans lequel m = 1 est préparé par les mêmes procédés.

Les composés II dans lesquels M³⁺ représente un autre cation métallique seront préparés de façon analogue par action du sel métallique, particulièrement le chlorure, ou de l'oxyde métallique sur une solution aqueuse d'un sel du ligand, comme décrit dans US 5,554,748 et les références qui y sont citées, ou dans Helv. Chim. Acta 69, 2067-2074 (1986), ou par échange de cation lorsque la stabilité relative des chelates le permet, notamment avec une résine échangeuse d'ions.

Pour les chelates avec un radioélément, on peut effectuer un échange radioisotopique par chauffage d'un chelate avec un sel minéral du métal radioactif dans l'eau ultrapure et élimination des cations non complexés par passage sur une résine complexante. On peut aussi libérer le cation métallique du chelate par un autre agent complexant en excès, de préférence donnant un chelate de Gd insoluble, tel que l'acide oxalique, pour obtenir le composé de formule II pur, avant de complexer un autre cation métallique.

Les composés II' dans lesquels m = 2 et R' est H, alkyl éventuellement hydroxylé, CH₂COOH et CH₂CONZ₁Z₂ peuvent être préparés à partir du cyclen monosubstitué par R' ou par un groupe protecteur éliminable, auquel cas l'introduction de R' a lieu après trialkylation avec 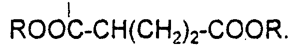 Parmi les procédés de monosubstitution, on peut citer la réaction de monoalkylation favorisée par le choix du solvant, décrite dans J. Org. Chem. 58, 3869-3876 (1993), celle avec complexation de 3 des atomes d'azote par des dérivés du bore ou du phosphore, décrite respectivement dans Tetrahedron Letters 32(5) 639-641 (1991) et Angew Chem. Int. Ed. 30(5) 560-561 (1991), ou celle avec formation d'un intermédiaire orthoamide décrite dans US 5,410,043. Lorsque R' est différent de H, il est préférable d'introduire d'abord R' au lieu d'effectuer directement la trialkylation du cyclen par un dérivé réactif de 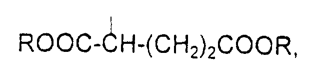 les dérivés dialkylés et tetraalkylés formés simultanément ne pouvant être éliminés par des opérations simples.

Les méthodes de purification des différents intermédiaires de synthèse et du produit final sont classiques mais doivent évidemment être adaptées à la nature chimique du produit, à sa masse moléculaire, à ses solubilités et à la viscosité de ses solutions. On peut mettre en oeuvre les méthodes de séparation chimiques (dissolution sélective, cristallisation) ou physiques par filtration sur membrane ou par un traitement par adsorbant, en suspension ou en colonne.

Notamment pour les composés III qui sont solubles en milieu aqueux, on pourra purifier par passage sur des résines échangeuses d'ions anionique et cationique ou par ultrafiltration, par diafiltration ou par osmose inverse sur une membrane convenablement choisie pour éliminer dans l'éluat les molécules de faible masse, les solvants et les sels.

Pour les composés II', il est préférable de ne pas effectuer un traitement pouvant modifier les proportions relatives des isomères et on peut purifier les esters intermédiaires dissous dans un solvant organique non miscible par agitation avec une phase aqueuse acide puis, après décantation, par mise en contact avec un adsorbant, tel que le Silica Gel, avant d'effectuer leur hydrolyse en milieu basique ou acide pour obtenir les acides II' à complexer.

Les chelates de formule I sont purifiés en général sous forme de sel alcalin soluble en milieu aqueux par l'une des méthodes précédemment citées, et notamment par traitement de leur solution aqueuse avec du noir de carbone ou de la silice silanisée.

Ces chelates de l'invention sont caractérisés par la présence sur le noyau central chelateur de bras latéraux comportant 3 ou 4 noyaux phényles successifs, réunis entre eux par des ponts de faible longueur, le dernier noyau phényle portant au moins un groupe hydrophile; malgré leur masse moléculaire élevée, et la présence de plusieurs noyaux phényles hydrophobes, ces composés sont solubles dans l'eau et biocompatibles et peuvent être administrés par voie intraveineuse chez l'homme.

En outre, et c'est une propriété très importante, ils franchissent peu les parois vasculaires contrairement aux composés commercialisés, ce qui permet d'obtenir lors de l'imagerie par résonance magnétique, un fort contraste entre les vaisseaux et les tissus avoisinants; cette propriété, associée à leur forte relaxivité, permet soit d'injecter au patient des doses de gadolinium complexé inférieures, soit de détecter des états pathologiques que l'on ne pouvait jusque là mettre clairement en évidence.

Les chelates paramagnétiques de l'invention peuvent être formulés pour une administration parentérale ou entérale, associés aux véhicules pharmaceutiques et excipients classiques dans ce domaine.

Pour une administration intraveineuse ou intraartérielle, les composés de l'invention se présentent en solution aqueuse stérile à la concentration de 0,001 à 0,5 mole/litre associés éventuellement à un agent stabilisant le pH vers 7, comme le tampon TRIS ou CO₂, à un agent isotonisant tel que mannitol, glycérol ou glucose de préférence à NaCl qui peut modifier la solubilité et la dispersion du chelate, ou à un autre complexant tel que l'EDTA.

Pour éliminer les endotoxines qui peuvent contaminer le produit fini, à la suite notamment de purification par chromatographie liquide, on effectue de préférence une ultrafiltration, éventuellement tangentielle à un volume sensiblement constant, sur des membranes dont la nature et le seuil de coupure sont fonction de la masse moléculaire du produit et la pression de la viscosité de la solution. Cette purification peut être réalisée aussi à certaines étapes intermédiaires sur des composés de plus petite taille, par microfiltration, notamment sur membrane chargée, ou par ultrafiltration. On peut aussi envisager des traitements par un absorbant, spécifique ou non des endotoxines tel que noir de carbone.

Pour une administration intravaginale les solutions aqueuses comportent de préférence un agent viscosifiant, telle qu'une gomme naturelle, un polysaccharide ou un dérivé de cellulose. Pour une administration rectale, le chelate sera formulé sous forme de suppositoire ou de solution visqueuse. Enfin, pour une administration orale, il pourra être présenté sous forme de gélules, de comprimés ou de sirop, préparés avec les excipients habituels. Des exemples de formulotions peuvent être trouvés dans Remington's for Pharmaceutical Science, 18e Edition (1990), Mack. Pub. Cy.

La dose unitaire et sa concentration sont fonction de la taille du patient, du type d'imagerie effectuée, mais aussi de la solubilité, de la viscosité en solution, de l'efficacité magnétique, de la perméabilité capillaire et de la pharmacocinétique du composé. Ils seront utilisés en imagerie par résonance magnétique en appliquant des techniques rapides ou non, pour étudier la perfusion, en particulier myocardique, cérébrale, rénale ou hépatique, pour des angiographies ou pour visualiser ou caractériser des anomalies de perméabilité, en particulier tumorale, inflammatoire et ischémique ou du cartilage.

Ces produits pourront aussi être utilisés en médecine nucléaire après introduction d'un atome radioactif, par exemple par échange isotopique des halogènes ou du cation complexé; une scintigraphie des zones à imager sera effectuée après administration des chelates de l'invention.

Dans ce qui suit, des exemples de composés de l'invention sont décrits ainsi qu'un procédé de préparation des chelates de gadolinium des composés de formule II dans laquelle 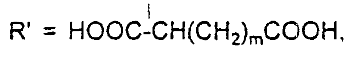 R' = H et R' = CH₂COOH, et des composés VI précurseurs des phényles.

Les produits isolés sont, selon les cas, caractérisés par leurs distances de rétention en chromatographie sur couches minces ou leurs temps de rétention (tᵣ) en chromatographie liquide haute performance (HPLC) ou en chromatographie d'exclusion stérique (CES). Leurs masses moléculaires ont été déterminées par spectrométrie de masse (electrospray).
A. Chelate de gadolinium de l'acide [1,4,7,10-tétraazacyclododécane]1,4,7,10-tétra(2-glutarique) (sel de sodium):
   - 1. Dans une solution de 25 g de 1,4,7,10-tétraazacyclododecane dans 280 ml d'acétonitrile, on introduit 30 g de carbonate de sodium puis 78 g de 2-bromoglutarate d'éthyle, préparé par exemple comme décrit dans Acta Chim. Acad. Sci. Hung 41(3) 331-6 (1964); le milieu est porté à sa température de reflux une journée au cours de laquelle on rajoute par 2 fois 78 g du dérivé bromé avec 30 g de carbonate de sodium. On filtre le précipité après refroidissement et on lave la phase organique avec de l'eau avant de l'extraire par une solution aqueuse diluée d'acide chlorhydrique. On extrait ensuite de la phase aqueuse amenée vers pH 3-4 avec du toluène.
      Le produit cherché est purifié par chromatographie sur silice en éluant avec du chlorure de méthylène, éventuellement en mélange avec de l'acétone.
   2. Hydrolyse des fonctions ester:
      On introduit 46 g de l'octaester en solution dans 52 ml d'éthanol dans 350 ml d'eau dans lesquels on a ajouté 50 g de NaOH, en perles.
      Après deux jours sous agitation à 80°C, on introduit dans la solution refroidie 500 ml de résine échangeuse cationique sous forme acide faible pour neutraliser puis après séparation de la phase solide, 500 ml de résine échangeuse anionique sous forme de base forte. La résine est séparée et introduite dans 500 ml de solution aqueuse d'acide acétique 6N; le produit final, passé en solution, est isolé sous forme d'une poudre par évaporation sous vide du solvant.
      HPLC: colonne 25 cm x 4,6 mm de silica gel Nucleosil® C18 100-5.
      Eluant No. 1: H₂SO₄ aqueux (O,1%) pendant 10 minutes puis avec 0 à 10% (V/V) de CH₃CN en 10 minutes: d = ml/min; T = 25°C;
      tᵣ = 5,4; 8,7; 10,2; 14 min (isomères) (CH₃COOH - tᵣ = 4,5 minutes).
   3. Complexation:
      Par l'oxyde de gadolinium: dans 30 ml d'une solution à un pH de 5,5 à 6 de 2 g de l'octaacide précédent, on introduit 0,47 g d'oxyde de gadolinium et on maintient le mélange à 80°C pendant 3 heures, au cours desquelles on ajuste le pH si nécessaire. Après refroidissement, à pH = 6,5 on introduit 2 g de résine Chelex® 100 commercialisée par Sigma sous forme Na⁺; après quelques heures de contact, on sépare la résine et on verse la solution sur 10 volumes d'éthanol pour précipiter le chelate.
      Par le chlorure de gadolinium: on amène à pH 6,5 par addition de NaOH aqueux (1N), le mélange de 6,5 g de l'octaacide et 3,5 g de Gd Cl₃, 6 H₂O dans 130 ml d'eau, et on le porte à 60°C durant 2 heures au cours desquelles le pH est maintenu à 6,5 par addition au total de 21 ml de NaOH aqueux 1N. Après quelques heures à température ambiante, on concentre jusqu'à 25 ml et on précipite le produit final dans 10 volumes de C₂H₅OH.
B. Chelate de gadolinium de l'acide 1,4,7,10-tétraazacyclododécane 1,4,7-tri-(2-glutarique) (formule II: 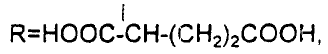 , R'= H, M= Gd):
   1. 10-benzyl 1,4,7,10-tétraazacyclododécane:
      30,5 g de chlorure de benzyle sont introduits goutte à goutte dans une solution de 50 g de 1,4,7,10-tétraazacyclododécane dans 500 ml de chloroforme. Après 24 heures d'agitation à température ambiante, le milieu réactionnel est filtré; le filtrat est concentré à sec. Le résidu est repris dans 350 ml d'eau et 75 ml de toluène puis après décantation de la phase toluénique, la phase aqueuse est extraite par 2 fois 200 ml de dichlorométhane. La phase organique est séchée sur sulfate de sodium puis concentrée à sec. On obtient 21 g du produit sous forme huileuse.
   2. 10-benzyl 1,4,7,10-tétraazacyclododécane 1,4,7-tri-(2-glutarate de méthyle):
      Une solution de 56,3 g de 2-bromoglutarate de méthyle dans 50 ml d'acétonitrile est introduite goutte à goutte dans une suspension de 18,7 g du produit précédent avec 24,7 g de carbonate de sodium dans 300 ml d'acétonitrile. Le milieu réactionnel est porté à sa température de reflux qui est maintenue durant 36 heures; après filtration à température ambiante le solvant est éliminé par évaporation et le résidu dissous dans le minimum d'acétate d'éthyle et purifié par chromatographie sur une colonne de silice en éluant par un mélange d'acétate d'éthyle et d'éther de pétrole (40/60 à 70/30 V/V). On obtient ainsi 20,7 g du produit sous forme d'huile.
   3. Acide 10-benzyl [1,4,7,10-tétraazacyclododécane] 1,4,7-tri-(2-glutarique):
      16,7 g de l'huile obtenue ci-dessus en solution dans 40 ml de méthanol sont introduits dans 125 ml d'eau dans lesquels 25 g de NaOH ont été dissous; le mélange est maintenu sous agitation à 70°C pendant 48 heures. On élimine ensuite le méthanol sous pression réduite avant d'introduire 400 ml de résine échangeuse de cations sous forme d'acide faible (Amberlite® - IRC 50 commercialisée par Rohm et Haas) puis, après séparation, d'anions sous forme de base forte (Amberlite® - IRA 458), le produit cherché s'étant fixé sur la résine anionique, il est isolé par élution avec 2 litres d'une solution aqueuse d'acide acétique 3N puis 2 litres de solution 6N.
      Après élimination des solvants on obtient 13,5 g de cristaux blancs, mélange d'isomères.
      HPLC: colonne No. 1: 25 cm x 4 mm Lichrospher® 100-RP18 - 5 µm (Merck, DE); Eluant No. 1.
      tᵣ = 28, 30 minutes (2 massifs d'isomères 75/25 en surface).
   4. Acide 1,4,7,10-tétraazacyclododécane 1,4,7-tri-(2-glutarique):
      13 g du composé précédent en solution dans 210 ml d'eau et 90 ml de méthanol sont hydrogénés durant 3 heures à température ambiante sous une pression de 28 x 10⁴ Pa en présence de palladium à 10% sur charbon humide à 50%. Après élimination du catalyseur et des solvants par évaporation, on obtient 11 g de poudre blanche.
      HPLC: colonne No. 1; Eluant No. 1.
      tᵣ = 8, 9 minutes (2 massifs d'isomères 75/25 en surface).
   5. Complexation:
      A 60°C, une solution de 1,6 g du composé précédent et 1 g de Gd Cl₃, 6 H₂O dans 30 ml d'eau est maintenue à pH 5 par addition de NaOH aqueux 3N, durant 4 heures. On concentre jusqu'à obtenir un volume de 10 ml et on précipite le résidu dans 100 ml d'éthanol. On isole par filtration 1,6 g du complexe de gadolinium cherché.
      HPLC: colonne No. 1; Eluant No. 1; tᵣ = 24, 26 minutes.
C. Chelate de gadolinium de l'acide 1,4,7,10-tétraazacyclododécane 1,4,7-tri-(2-glutarique) 10-acétique:
   1. 1 g de l'acide obtenu à l'étape 4 de la préparation B sont mis en solution dans 8 ml d'eau, avec 270 mg d'acide bromoacétique et le mélange est maintenu 3 heures à 70°C et pH 10 contrôlé par addition de NaOH aqueux 1N. On ajoute ensuite à température ambiante 100 ml d'eau et 10 ml de résine Amberlite® IRC 50; après 1 heure de contact la résine est séparée et on introduit dans le milieu 50 ml de résine Amberlite® IRA 458. Après 12 heures, la résine est séparée et introduite dans une colonne. Le produit cherché en est extrait par élution avec 500 ml d'une solution aqueuse d'acide acétique 1N. On obtient ainsi 0,8 g de cristaux blancs.
      HPLC: colonne No. 2: 25 cm x 4 mm Symetry® - RP 18 - 5 µm (Waters); Eluant No. 1; tᵣ = 4 minutes.
   2. Complexation:
      On introduit dans 13 ml d'eau, 0,45 g de GdCl₃, 6 H₂O et 0,75 g du composé précédent; le pH est amené à 6 par addition d'une solution aqueuse de NaOH(1N); le milieu réactionnel est alors maintenu à 60°C durant 5 heures au cours desquelles on ajuste plusieurs fois le pH par addition de la solution de NaOH. Après refroidissement, le milieu est introduit dans 130 ml d'éthanol et le précipité est isolé pour donner 0,75 g de chelate.
D. N,N'-(2,3,4,5-tétrahydroxypentyl) N,N'-[2-(hydroxyéthoxy)éthyl] 2,4,6-triiodo 5-(glycylamino)isophtalamide:
   1. 5-[2-(hydroxyéthoxy)éthylamino]pentane 1,2,3,4-tétraol:
      On dissout 84 g de 2-aminoéthoxyéthanol et 150 g de D-xylose dans 2,5 l de méthanol; le mélange est hydrogéné à température ambiante sous une pression de 6 x 10⁵ Pa, en présence de 50 g de palladium sur charbon à 10%. Le catalyseur est éliminé par filtration et le solvant distillé. Le résidu, dissous dans le minimum d'eau, est chromatographié sur 1OO ml de résine Amberlite® IRN 77 (H⁺) en éluant avec une solution aqueuse de NH₄OH puis sur 1 kg de gel de silice Geduran® SI60 pour donner 170 g d'aminoalcool sous forme d'huile brune.
      CCM: CH₃OH/NH₄OH à 25% - (7/3 - V/V) - Rf = 0,3.
      RMN ¹³C: (DMSO- d6 - 50,3 MHz); δ (ppm): 51,4; 48,9 (2 x CH₂NH); 62,9; 60,5 (2x CH₂OH); 71,9; 71,2; 70,6 (3 x CHOH); 72,3; 69,7 (CH₂OCH₂).
      On peut préparer de la même manière le dérivé de l'arabinose ou du ribose.
   2. N,N'-(2,3,4,5-tétrahydroxypentyl) N,N'-[2-(hydroxyéthoxy)éthyl] 2,4,6-triiodo 5-(phtalamidoacétamido)isophtalamide:
      On dissout 30 g de l'aminoalcool précédemment préparé dans 100 ml de diméthylacétamide, à 45°C, puis on introduit 22 g de dichlorure du dérivé d'acide isophtalique et 12 g de triéthylamine et on laisse le milieu sous agitation à cette température durant 24 heures. On isole le précipité formé à température ambiante et on élimine le solvant par distillation sous vide.
      Le résidu en solution dans 50 ml d'eau est élué sur 100 ml de résine Amberlite® IMAC HP 1110 (H⁺); après concentration de la solution jusqu'à 10 ml, on la verse dans 1500 ml d'alcool isopropylique et on isole le précipité formé après 24 heures.
   3. Libération de l'amine:
      On introduit à 80°C dans une solution de 40 ml d'eau et 2,7 ml d'hydrate d'hydrazine, 42 g du produit préparé ci-dessus en solution dans 60 ml d'eau. Après 5 heures d'agitation à cette température, on filtre puis acidifie le milieu jusqu'à pH 1 par addition d'acide chlorhydrique concentré. Le précipité formé est séparé et le filtrat concentré. Le résidu en solution dans 30 ml d'eau est chromatographié sur 30 ml de résine Amberlite® IMAC HP 111E (H⁺) puis sur 55 ml de résine Amberlite® IRA 67 (OH⁻). L'éluat concentré est fixé sur 350 ml de résine Amberlite® 252 Na (H⁺), d'où il est élué par une solution aqueuse de NH₄OH (2N). p = 20 g.
      HPLC: colonne No. 1; Eluant No. 2: H₂O/CF₃COOH (pH 3,4) avec gradient d'acétonitrile 95/5 à 50/50 V/V en 50 minutes;
      tᵣ = 10 - 20 minutes (mélange d'isomères).
E. N,N'-[bis(2,3,4,5,6-pentahydroxyhexyl)]2,4,6-tribromo 5-(glycyl amino)isophtalimide:
   1. Acide 5-amino 2,4,6-tribromo isophtalique:
      On introduit lentement 156 g de brome dans 300 ml d'une solution aqueuse de 50 g d'acide 5-aminoisophtalique et 55 ml d'acide chlorhydrique à 37%. Après 1 nuit d'agitation on neutralise l'excès de brome par addition d'une solution aqueuse de bisulfite de sodium avant d'isoler le précipité. Rendement: 90%.
   2. Acide 5-(phtalimidoacétamido) 2,4,6-tribromo isophtalique:
      On introduit lentement 27 ml de chlorure de thionyle dans une solution de 69 g de N-phtaloylglycine, dans 200 ml de diméthylacétamide à 10°C, puis après 2 heures d'agitation on introduit vers 15-20°C, 100 g de l'acide obtenu précédemment. Après 1 nuit à température ambiante, le mélange est versé dans 800 ml d'eau chaude. On isole ainsi 140 g de produit final.
   3. Chlorure du diacide précédent:
      On introduit lentement à 18°C dans une solution de 100 g du diacide dans 300 ml de dioxanne et 50 ml de diméthylformamide, 70 ml de chlorure de thionyle. Le précipité jaune formé après 3 journées d'agitation à température ambiante est filtré et lavé avec de l'oxyde de méthyle et de t-butyle. On obtient ainsi 70 g de solide beige.
   4. N,N'[-bis(2,3,4,5,6-pentahydroxyhexyl)]2,4,6-tribromo 5-(phtalimidoacétamido)isophtalamide:
      On dissout 150 g de disorbitylamine dans 600 ml de N-méthylpyrrolidone à 80°C et on introduit dans le milieu à 60°C, 16 g de carbonate de sodium sec, puis 96 g du chlorure d'acide précédent. Après 1 heure d'agitation à cette température et 16 heures à température ambiante, on élimine le précipité et on verse la solution dans 1,6 I d'isopropanol. Le précipité isolé pèse 200 g.
   5. Hydrazinolyse:

   200 g du produit précédent et 17 ml d'hydrate d'hydrazine sont introduits dans 400 ml d'eau à 70°C. Après 3 heures d'agitation on acidifie jusqu'à pH 4 par addition d'acide chlorhydrique 6N à température ambiante. On élimine alors le précipité formé et on neutralise le filtrat par addition d'une solution aqueuse de NaOH 1N. L'hydrazine en excès est éliminée par osmose inverse. La solution résiduelle est traitée par 10 ml de résine cationique forte puis 65 ml de résine anionique faible.
   Le produit final est alors extrait de la solution par fixation sur une résine cationique forte sous forme H⁺, d'où elle est éluée par une solution aqueuse diluée de NaCl (0,1 M). p = 80 g.
   HPLC: colonne No. 1; Eluant No. 2; tᵣ = 7 minutes environ.
F. N,N'-[bis(2,3,4,5,6-pentahydroxyhexyl)]2,4,6-triiodo 5-(glycylamino) isophtalimide:
   1. Chlorure de l'acide 5-(phtalamidoacétamido) 2,4,6-triiodoisophtalique:
      On introduit 149 ml de SOCl₂ dans une solution de 335 g de N-phtaloylglycine dans 1 I de N-méthylpyrrolidone à 10°C puis après 3 heures 700 g de dichlorure d'acide 2,4,6-triiodo 5-aminoisophtalique. Après 3 jours d'agitation à température ambiante, le milieu est versé sur 4,5 l d'éthanol aqueux 2/1 V/V). On amène le pH à 5 par addition de triéthylamine avant d'isoler le précipité formé, qui peut être purifié par lavage à l'isopropanol. p = 850 g.
   2. Amidification par la disorbitylamine H-N[CH₂(CHOH)₄CH₂OH]₂:
      En opérant comme pour le composé analogue tribromé, on obtient le produit avec 85% de rendement.
   3. Hydrazinolyse:
      420 g du phtalimide triiodé précédent en solution dans 1 l d'eau et 300 ml d'hydrate d'hydrazine sont maintenus plusieurs heures à 80°C. Après acidification du milieu à pH 3,6 on élimine l'excès d'hydrazine par chromatographie sur une résine échangeuse de cations (H⁺) telle que IMAC® HP111E commercialisée par Rohm et Haas, puis les chlorures avec une résine échangeuse d'anions (OH⁻).
      Le produit final peut être purifié par chromatographie sur résine échangeuse de cations (H⁺ forte) et précipité à partir de sa solution aqueuse dans l'éthanol. Rendement 50%.
      HPLC: colonne No. 1;
      Eluant No. 4: CH₃CN/P.I.C.® B8 (0,05 M) (Waters) 15/85; débit 1 ml/min.
      tᵣ = 3 minutes.
G. 1-deoxy 1-[(2,3-dihydroxypropyl)amino] D-galactitol 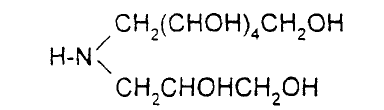
   On maintient 27 g de 3-aminopropane 1,2-diol et 50 g de D-galactose dans 140 ml de méthanol sous agitation à 40°C pendant 16 heures. On ajoute alors 60 ml d'eau et on effectue, en présence de 7 g de palladium sur charbon (à 10%) l'hydrogénation de l'imine durant 7 heures à 60°C sous une pression de 20 x 10⁵ Pa d'hydrogène.
   Le catalyseur est alors séparé par filtration sur Célite®. Après élimination du solvant sous pression réduite, le résidu, dissous dans le minimum d'eau (50 ml), est introduit lentement dans 600 ml d'isopropanol. Le précipité formé est isolé. F = 132°C, rendement 90%.
   On peut aussi avant la précipitation dans l'isopropanol, chromatographier le produit brut, en solution dans 200 ml d'eau sur 500 ml d'une résine échangeuse d'ions sous forme acide sulfonique en éluant par une solution aqueuse de NH₄OH dilué.
   Dans ces conditions, l'aminoalcool est un mélange pratiquement 50/50 des deux diastéréoisomères.
   Par recristallisation, précipitation fractionnée ou mise en suspension, on peut enrichir le mélange en l'un ou l'autre des isomères.
   Ainsi, par traitement au méthanol à sa température de reflux (25 g de l'aminoalcool dans 500 ml du solvant) on obtient un mélange qui contient un excès de l'un des isomères: 65% de l'isomère ayant le plus grand temps de rétention d'après les surfaces mesurées sur les chromatogrammes obtenus dans les conditions suivantes:
   Chromatographie en phase gazeuse (dérivé totalement trifluoroacétylé par action de l'anhydride trifluoroacétique à 60° C);
   Appareil: Varian Star 3400;
   Colonne: DB 1701 de J & W (0,25 µm - 30 m x 0,25 mm);
   Gaz vecteur He - T injecteur (split 1/40^{e}) = 290°C;
   T colonne = 150°C à 280°C (5°C/mn) - Volume: 1 µl;
   tᵣ: 14,5 et 14,9 minutes (5,6 mn pour l'aminopropanediol de départ).
   RMN ¹³C (200 MHz - DMSO d6 - Réf DMSO - T = 30°C)
   δ (ppm): 71,7; 71,6; 70,5; 70,1; 69,5; 68,5; 68,2 - (CHOH) 64,6; 63,2; (CH₂OH) - 53,4; 53; 52,9 (NCH₂).
   On peut aussi obtenir chaque diastéréoisomère en condensant le D-galactose sur l'énantiomère pur de l'aminopropanediol ou par action des énantiomères du glycidol sur la N-(2,3,4,5,6-pentahydroxyhexyl)benzylamide comme suit:
   a) N-benzyl N-(2,3,4,5,6-pentahydroxyhexyl)amine:
      On dissout dans 230 ml de méthanol 29 g de D-galactose, 17 ml de benzylamine et on maintient le mélange à 50°C pendant 7 heures sous une pression d'hydrogène de 8x10⁵ Pa après addition de 5 g de charbon palladié à 5%.
      Après l'hydrogénation, on introduit à 40°C, une solution aqueuse d'acide chlorhydrique jusqu'à pH acide dans le milieu réactionnel.
      On filtre le mélange sur Célite® à température ambiante pour éliminer le catalyseur, et après concentration partielle, on introduit une solution aqueuse de NaOH 5N jusqu'à pH basique. Le précipité formé est isolé et recristallisé dans l'éthanol.
      RMN ¹³C (200 MHz - DMSO d6 - Réf DMSO - T = 30°C)
      δ (ppm): 140,8 (C CH₂N) - 128,8; 128,4; 126,9 (phényl) - 72; 70,6; 69,9; 68,8; (CHOH) - 63,2 (CH₂OH) - 53,1; 52,4 (CH₂NH).
   b) N-benzyl N-(2,3-dihydroxypropyl)(2,3,4,5,6-pentahydroxyhexyl)amine:
      On dissout 6,5 g de l'amine secondaire obtenue dans l'étape précédente dans 200 ml de méthanol à 60°C, puis on introduit dans la solution à cette température 2,5 g de glycidol (racémique ou énantiomère pur) et on maintient le mélange sous agitation 24 heures. On élimine ensuite le solvant par distillation sous pression réduite, et on chromatographie le résidu après dissolution dans 200 ml d'eau sur une colonne de résine Amberlite® IMAC 110 sous forme H⁺ en éluant avec une solution aqueuse de NH₄OH diluée (O,1%). On obtient ainsi 5,5 g de l'amine.
   c) N-(2,3N-(2,3-dihydroxypropyl) N-(2,3,4,5,6-pentahydroxyhexyl)amine:
      2 g de l'amine tertiaire obtenue dans l'étape précédente en solution dans 30 ml d'eau sont hydrogénés à 45°C sous pression d'hydrogène de 100x10⁵ Pa pendant 5 heures en présence de 0,6 g de charbon palladié à 10%. La solution est ensuite filtrée sur Célite® pour éliminer le catalyseur et concentrée sous pression réduite. Le résidu peut être recristallisé dans l'éthanol.
      Lorsque l'on condense le (S)-glycidol on obtient l'aminoalcool diastéréoisomère dont le temps de rétention dans les conditions de la chromatographie en phase gazeuse précédente est le plus long.
      A partir du D-glucose et du D-mannose, on obtiendra en appliquant ces procédés des diastéréoisomères de ces aminoalcools, alors que d'autres aminoalcools pourront être obtenus à partir d'aminoalcools primaires ou de sucres différents.
H. Chelate de gadolinium de l'acide (1,4,7,10-tétraazacyclododécane) 1,4,7,10-tétra(2-succinique):
   1. Condensation de l'acétylène dicarboxylate de benzyle sur les 4 atomes d'azote du cyclen:
      On verse goutte à goutte à 20°C, 23 g d'acétylène dicarboxylate de benzyle, préparé comme décrit dans J.C.S. Perkin I, p. 2024-2029 (1973), en solution dans 25 ml d'acétonitrile sur une solution de 2,7 g de cyclen dans 50 ml d'acétonitrile; Après 3 heures d'agitation à 50°C, on élimine le solvant sous pression réduite et on purifie par chromatographie sur silice en éluant avec un mélange dichlorométhane/méthanol (98/2 - V/V) pour isoler 19 g de solide jaune.
   2. Réduction de l'énamine:
      On introduit par portions sous agitation, 7 g de cyanoborohydrure de sodium dans une solution de 19 g du solide dans 500 ml d'acétonitrile et 50 ml d'acide acétique et on maintient le mélange une nuit sous agitation à température ambiante. Après élimination du solvant sous pression réduite, le résidu huileux est mis en solution dans un mélange dichlorométhane/méthanol (99/1) et filtré sur silice. Après évaporation du solvant, on isole 19 g d'huile jaune.
   3. Débenzylation:
      8 g de l'octaester obtenu, dans un mélange d'eau/méthanol (175 ml/75 ml) sont hydrogénés pendant 6 heures en présence de 4 g de palladium sur charbon à 10% (pression: 3,5x10⁵ Pa). Après séparation du catalyseur par filtration et élimination du solvant par distillation, on obtient 3 g d'octaacide sous forme de poudre blanche.
      HPLC: Colonne No. 1; Eluant No. 1; tᵣ = 18 à 19 minutes.
   4. Complexation:
      1 g de l'octaacide obtenu est mis en suspension dans 20 ml d'eau, le pH est amené à 5,5 par addition d'une solution aqueuse de NaOH 1N et 0,285 g de Gd₂O₃ sont ajoutés. Après 4 heures d'agitation à 70°C du milieu réactionnel dont le pH est maintenu entre 5,5 et 6,5 par addition d'une solution aqueuse de HCI 1N, il est filtré et le filtrat concentré à sec pour donner le sel de sodium du chelate.

### Exemple 1.

Chelate de gadolinium de formule I dans laquelle m = 2,
R' = 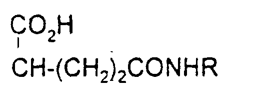 R = 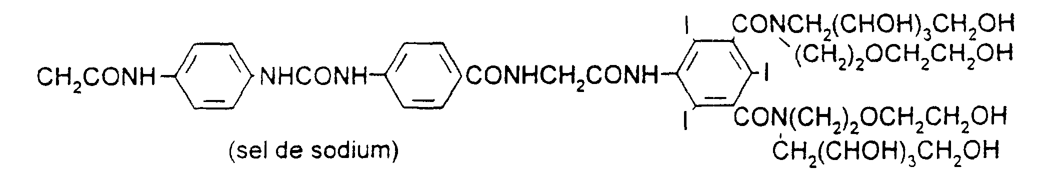
1. Acide 4-[N'-(4-nitrophényl)uréido]benzoïque:
   A 10° C, on introduit sous agitation dans 80 ml de tétrahydrofurane 24,6 g de 4-nitrophénylisocyanate, puis lentement 20,5 g d'acide 4-aminobenzoïque en solution dans 70 ml de tétrahydrofurane. On laisse revenir le mélange à température ambiante et on poursuit l'agitation 1 heure avant d'isoler le précipité formé. p = 45 g. HPLC: colonne No. 1; Eluant No. 2: H₂O/CF₃COOH; pH = 3,4 avec gradient d'acétonitrile (95/5 à 50/50 - V/V en 50 min); débit 1 ml/min; tᵣ = 44 minutes.
2. Acide 4-[N'-(4-aminophényl)uréido]benzoïque:
   22 g du produit ci-dessus et 37 ml de solution aqueuse de NaOH 1N sont introduits dans 280 ml d'eau avec 3 g de palladium sur charbon (5%). On maintient le milieu sous une pression d'hydrogène de 0,6 MPa pendant 6 heures à 65°C. Après retour à température ambiante, on amène le pH à 10 et on filtre le catalyseur sur Celite® avant d'acidifier jusqu'à pH 5,3 par addition d'une solution aqueuse de HCI 6N; le précipité obtenu est lavé à l'acétone. p = 13,2 g.
   HPLC: colonne No. 1; Eluant No. 2; tᵣ = 33 minutes.
3. Acide 4-[N'[4-(phtalimidoacétamido)phényl]uréido]benzoïque:
   On dissout 5,8 g d'acide phtalimidoacétique dans 26 ml de diméthylacétamide à 10°C et on introduit, en maintenant la température, 2 ml de chlorure de thionyle, puis, après 2 heures d'agitation, 7 g du composé obtenu à l'étape précédente.
   Après 12 heures à température ambiante, le milieu réactionnel est versé dans 250 ml d'eau et le précipité formé lavé jusqu'à neutralité avec de l'eau à 25°C puis 95°C. p = 10,4 g.
   HPLC: colonne No. 1; Eluant No. 2; tᵣ= 40 minutes.
4. N,N'-(2,3,4,5-tétrahydroxypentyl)N-N'-[2-(hydroxyéthoxy)éthyl] 2,4,6-triodo 5-[4-[N'-(4-phtalimidoacetamidophényl)ureido]benzoyl glycylamino]isophtalamide:
   On dissout dans 40 ml de diméthylacétamide 4 g du composé obtenu ci-dessus avec 1,7 g d'HOBT, H₂O et 9,3 g de N,N'-bis-(2,3,4,5-tétrahydroxypentyl)N,N'-bis(hydroxyéthoxyéthyl) 2,4,6-triodo 5-(glycylamino) isophtalamide puis 2,4 g d'EDCI, HCI à 0°C. Après 1 nuit d'agitation à température ambiante le milieu est versé dans 300 ml de dichlorométhane. Le précipité isolé est lavé à l'éther diéthylique.
   HPLC: colonne No. 1; Eluant No. 2; tᵣ= 30-33 minutes (mélange d'isomères).
5. Elimination du groupe phtalimido: produit de formule III dans laquelle Z = CH₂CONH; Z' = NHCONH; Z" = CONHCH₂CONH;
   R₁ = R₃ = R₅ = I; 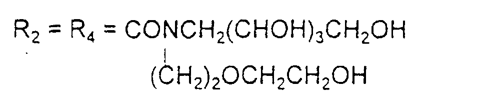
   Dans 45 ml d'eau, on introduit à 80°C, 11,5 g du composé précédent puis 1,12 ml d'hydrate d'hydrazine (0,023 mol). Après 2 heures 45 à 80°C, on refroidit à O°C et acidifie jusqu'à pH 1 par addition de HCI aqueux 12N. Le mélange est ensuite filtré sur Celite® à température ambiante puis filtré sur 16 ml de résine cationique (H⁺) IMAC® HP111E et 80 ml de résine anionique faible (OH⁻) IMAC® HP661, commercialisées par Rohm et Haas.
   Après une dernière purification par filtration sur silice silanisée, on obtient 4,6 g du produit final.
   HPLC: colonne No. 1; Eluant No. 2; tᵣ = 24-27 minutes (mélange d'isomères).
6. Condensation des produits A et III
   On introduit à 40°C dans 20 ml d'une solution aqueuse à pH6, 4,5 g de l'amine ci-dessus et 0.63 g du chelate A, 0,057 g de NHS (sel de sodium)et 0,76g d'EDCI, HCI. Après 15 minutes le mélange est versé dans l'eau et le précipité isolé. Après élimination des produits de départ on obtient le composé de formule I.
   Chromatographie d'exclusion stérique: (CES).
   Conditions No. 1: effectuée sur une succession de 4 colonnes (30 cm x 8 mm) commercialisées par Shodex (JP) sous les références OH Pak SB-HQ, contenant du gel de polyhydroxymétacrylate, dont les limites d'exclusion, déterminées avec du pullulan, sont successivement 10⁶ Kdaltons (SB-804); 10⁵ Kdaltons (SB-803); 10⁴ Kdaltons (SB-802-5); 10⁴ Kdaltons (SB-802-5).
   Eluant: NaCI aqueux (0,16 M)/acétonitrile (70/30-V/V); débit 0,8 ml/min.
   T = 30°C; tᵣ: 38 minutes (50-53 min pour l'amine de départ).
   HPLC: colonne No. 3, 25 cm x 4,6 mm Platinum EPS C18 100 Å; 5 µM (Alltech);
   Eluant No. 3: eau/CH₃CN de 98/2 à 60/40 - V/V en 50 min; débit: 1 ml/min;
   T = 25° C; tᵣ = 26 minutes.

### Exemple 2.

Chelate de gadolinium de formule I dans laquelle m = 2
R' = 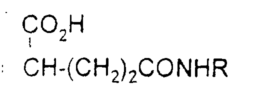 R = 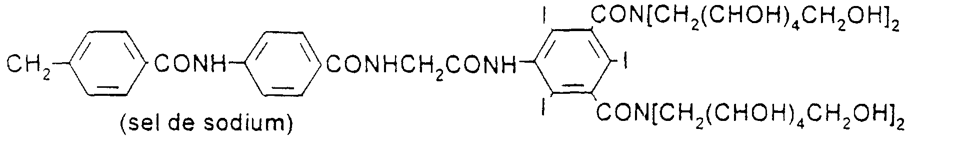
1. Acide 4-[4-(phtalimidométhyl)benzamido]benzoïque;
   On introduit dans une solution préparée avec 25 g d'acide 4-aminométhylbenzoïque, 18 g de Na₂CO₃ et 165 ml d'eau, 37 g de N-carbethoxyphtalimide. Le précipité formé est isolé puis mis en suspension dans 60 ml de diméthylacétamide dans lequel on introduit lentement à 10°C, 4,7 ml de chlorure de thionyle. Après 3 heures à cette température, on introduit 9 g d'acide 4-aminobenzoïque et on maintient le milieu 12 heures à température ambiante, avant de le verser dans 600 ml d'eau. Le précipité formé est isolé.
   HPLC: colonne No. 1; Eluant No. 2; tᵣ= 48 minutes.
2. N,N'-bis(2,3,4,5,6-pentahydroxyhexyl) 2,4,6-triiodo 5-[4-(4-amino méthylbenzamido)benzoylglycylamino]isophtalamide:
   a) On agite jusqu'à dissolution 160 ml de diméthylacetamide avec 39 g de 5-glycylamino N,N'-bis(2,3,4,5,6-pentahydroxyhexyl) 2,4,6-triiodo isophtalamide, 4,6 ml de triéthylamine, 5,7 g d'HOBT, H₂O et 12 g du produit obtenu ci-dessus, avant de refroidir à O°C et d'ajouter 8 g d'EDCI, HCI. En fin de réaction le milieu est versé sur 1000 ml de dichlorométhane. Le précipité isolé est purifié par précipitation dans le méthanol à partir de sa solution aqueuse. p = 43 g.
   b) Hydrazinolyse:
      25 g de phtalimide précédemment obtenus sont dissous dans 50 ml d'eau à 80°C avant d'ajouter 2,2 ml d'hydrate d'hydrazine. Après 3 heures d'agitation, le milieu est refroidi et acidifié jusqu'à pH 1 par addition d'HCI 12N. Le milieu est alors filtré sur Célite® puis purifié par chromatographie sur résine cationique faible (H⁺) puis sur résine anionique faible (OH⁻).
      On isole ainsi 15 g du produit cherché.
      HPLC: colonne No. 1; Eluant No. 2; tᵣ= 19 minutes.
c) Condensation sur le chelate de gadolinium A:
   15 g de l'amine ainsi obtenue et 1,9 g du chelate A (sel de sodium) sont dissous dans 40 ml d'eau. Après acidification jusqu'à pH 6 par addition de HCI aqueux 1N, on introduit à 40°C 2,3 g d'EDCI, HCl. Après 2 heures d'agitation, le milieu est introduit dans 400 ml d'éthanol; le précipité formé, mis en solution aqueuse, est filtré sur noir de carbone Norit. On obtient ainsi 8,5 g de produit pur.
   CES: conditions No. 1; tᵣ = 36 minutes.
   HPLC: colonne et éluant No. 3; tᵣ = 23 minutes.

### Exemple 3.

Chelate de gadolinium de formule I dans laquelle m = 2
R' = 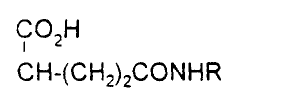 R = 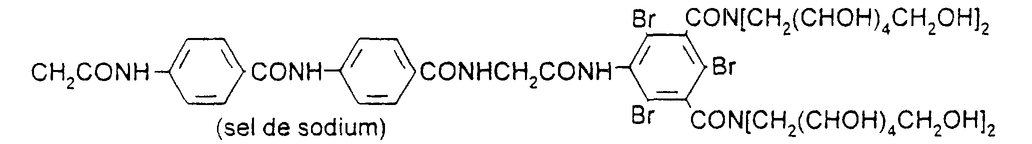
a) Acide 4-[4-nitrobenzamido]benzoïque:
   Peu à peu, on introduit 100 g de chlorure d'acide 4-nitrobenzoïque dans 74 g d'acide 4-aminobenzoïque et 360 ml de diméthylacétamide, en maintenant la température à moins de 25°C. Après 24 heures d'agitation, on ajoute à 10°C, 500 ml de chlorure de méthylène, pour précipiter le produit cherché. Après lavage à l'eau et séchage, on isole 145 g de produit.
b) Acide 4-(4-aminobenzamido)benzoïque:
   On soumet une suspension de 136 g d'acide précédent dans 1,8 litre d'eau dans laquelle on a ajouté 240 ml de solution aqueuse de NaOH 1N et 14 g de palladium sur charbon (10%) à une pression d'hydrogène de 0,6 MPa pendant 4 heures.
   Le pH de la suspension finale est alors amené vers 10 avant filtration sur Celite® pour éliminer le catalyseur. Le précipité formé lors de l'acidification du filtrat jusqu'à pH 5,3 est isolé et séché. p = 106 g; F > 260°C.
c) Acide 4-[4-(phtalimidoacétamido)benzamido]benzoïque:
   On introduit dans une solution de 90 g d'acide phtalimidoacétique dans 400 ml de diméthylacétamide à 10°C, 32 ml de chlorure de thionyle goutte à goutte puis, après 3 heures d'agitation, 105 g de l'aminoacide précédemment obtenu à une température inférieure à 20°C.
   Après 12 heures d'agitation, le milieu est versé dans 4 litres d'eau et le précipité isolé, lavé à l'eau chaude. Poids après séchage: 176 g. F > 260°C.
d) Chlorure de l'acide précédent:
   On introduit 2,5 ml de chlorure de thionyle dans 10 g de l'acide en suspension dans 50 ml de dioxane, et 1 ml de diméthylformamide et on maintient le mélange sous agitation à 50°C pendant 5 heures.
   Après addition d'un volume d'éther diisopropylique, on isole 10 g de précipité.
   On peut aussi mettre l'acide en suspension dans le toluène avec du chlorure de tricaprylylméthylammonium comme catalyseur.
e) N,N'-bis(2,3,4,5,6-pentahydroxyhexyl) 2,4,6-tribromo 5-(4-[4-(phtalimidoacetamido)benzamido]benzoylglycylamino)isophtalamide:
   Une solution de 2,25 g de chlorure d'acide avec 5 g de N,N'-bis (2,3,4,5,6-pentahydroxyhexyl) 2,4,6-tribromo 5-(glycylamino)isophtalamide et 0,7 ml de triéthylamine dans 25 ml de diméthylacétamide ou de N-méthylpyrrolidone est maintenue 12 heures sous agitation puis versée dans 60 ml d'éthanol. On isole ainsi 6,2 g de précipité.
   HPLC: colonne No. 1; éluant No. 2; tᵣ = 27-35 min. (mélange d'isomères).
f) Hydrazinolyse:
   On introduit une solution de 0,6 ml d'hydrate d'hydrazine dans 10 ml d'eau dans une solution de 10 g du phtalimide précédent dans 40 ml de diméthylacétamide à 80° C. Après 3 heures d'agitation à cette température, le mélange refroidi est versé dans 125 ml d'éthanol. On isole 9 g de précipité qui est purifié par traitement de sa solution aqueuse par une résine anionique forte (OH⁻) puis cationique faible (H⁺). p = 8 g.
   On peut aussi acidifier le milieu réactionnel pour séparer le phtalylhydrazide précipité et éliminer le solvant et les molécules de faible masse par ultrafiltration avant une précipitation finale dans l'éthanol aqueux. HPLC: colonne No. 1; Eluant No. 2 mais 90/10 V/V sans gradient; tᵣ = 28-35 min.
g) Couplage sur le chelate A pour obtenir le composé de formule I:

On dissout dans 135 ml d'eau 5 g du composé obtenu à l'étape (f) avec 0,65 g du chelate A (sel de sodium); le pH du milieu est abaissé jusqu'à 6 par addition d'HCI aqueux 1N avant d'introduire à 40° C 0,8 g d'EDCI, HCI dans le milieu. Après 2 heures d'agitation et retour à température ambiante, le milieu est versé dans 135 ml d'éthanol.

On peut aussi introduire 1,6 g du chelate A (sel de sodium) dans 25 ml de solution aqueuse de 10 g de l'amine obtenue en f puis à pH 6,5, successivement 18 ml de dioxanne, 1,4 g d'EDCI et 0,08 g d'HOBT. Après 2 heures à température ambiante, on verse le milieu réactionnel dans 100 ml d'éthanol et on isole le précipité. Redissous dans 100 ml d'eau, il est traité deux fois par 1 g de noir de carbone avant d'être reprécipité dans 3 volumes d'éthanol.

Le précipité formé est purifié par traitement avec 5 ml de résine échangeuse d'ions Chelex 100 (Na⁺) commercialisée par Sigma, puis par dia-ultrafiltration sur membrane de seuil de coupure 5 KD, à pH 7. p = 4 g.
CES: Conditions No. 1; tᵣ = 34 minutes.
HPLC: colonne et éluant No. 3; tᵣ = 23 minutes.

### Exemple 4.

Chelate de gadolinium de formule I dans laquelle m = 2
R' = H et R =

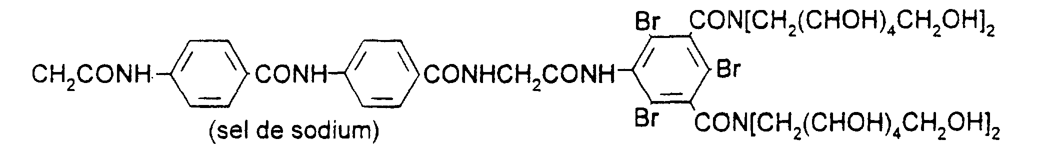

On maintient 5 heures à 40°C, sous agitation, 0,3 g du complexe de gadolinium de l'acide 1,4,7,10-tétraazacyclododécane 1,4,7-triglutarique préparé selon (B), 2 g de l'amine préparée à l'exemple 3 (f) et 0,3 g d'EDCI, HCl dans 5 ml d'eau, en maintenant le pH à 7 par addition d'une solution aqueuse de HCl 1N. Le milieu est versé dans 50 ml d'éthanol et le précipité formé est isolé. Il est purifié en solution dans 120 ml d'eau par ultrafiltration sur une membrane de 3 KD de seuil de coupure pour éliminer les produits de départ n'ayant pas réagi. Après agitation sur noir de carbone et filtration, la solution est amenée à sec pour donner 1 g de solide blanc.
CES: Condition No. 1; tᵣ= 36,8 minutes.
HPLC: colonne et éluant No. 3; tᵣ = 31 minutes.

### Exemple 5.

Chelate de gadolinium de formule I dans laquelle m = 2
R' = CH₂COOH et R =

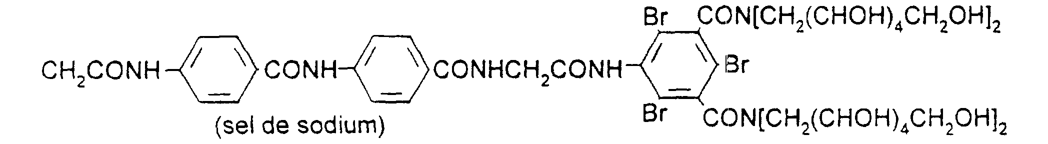

En appliquant le même mode opératoire que pour l'exemple 4, on obtient 0,8 g de produit final à partir de 0,3 g de chelate de départ C et 2 g de l'amine préparée à l'exemple 3 f.
CES: Conditions No. 1; tᵣ = 35,7 minutes.
HPLC: colonne et éluant No. 3; tᵣ = 22 minutes.

### Exemple 6.

Chelate de gadolinium de formule I dans laquelle m = 2
R' = 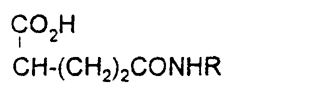 R = 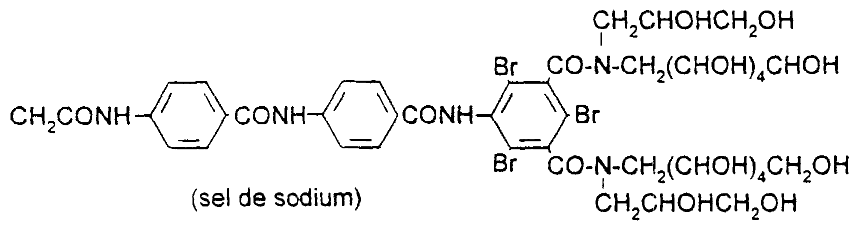
1. Acide 5-[4-(4-aminobenzamido)benzamido] 2,4,6-tribromo isophtalique:
   (a) Chlorure de l'acide 4-(4-nitrobenzamido)benzoïque:
      On maintient 8 heures à la température de reflux dans 250 ml de chlorure de thionyle et 0,1 ml de diméthylformamide, 70 g de l'acide. L'excès de chlorure de thionyle est distillé sous pression réduite et le résidu est versé sous agitation sur 1,5 I d'acétate d'éthyle et 500 g de glace pilée.
      Le produit final est extrait dans la phase organique, celle-ci est lavée avec de l'eau, une solution aqueuse de bicarbonate de sodium puis séchée et concentrée. p = 64 g.
   (b) Acide 5-[4-(4-nitrobenzamido)benzamido] 2,4,6-tribromo isophtalique:
      On maintient 18 heures à la température de reflux une solution de 64 g du chlorure d'acide avec 67 g d'acide 5-amino 2,4,6-tribromo isophtalique dans 170 ml de dioxane. Le solvant est évaporé et le résidu, après lavage par 300 ml d'acétate d'éthyle chaud, est dissous dans 600 ml d'eau avec une quantité suffisante de NaOH 5N pour obtenir un pH 7; après lavage à l'acétate d'éthyle et acidification, on isole le produit cherché, précipité. p = 73 g.
   (c) Réduction:

   19 g du produit précédent en solution aqueuse à pH 6 sont maintenus 7 heures sous une pression d'hydrogène de 0,5 MPa en présence de 2 g de platine sur charbon type 156 (Johnson Matthey). Après filtration, la solution est évaporée. p = 15 g.
2. N,N'-(2,3dihydroxypropyl) N,N'-(2,3,4,5,6-pentahydroxyhexyl) 2,4,6-tribromo 5-[4-(4-glycylaminobenzamido)benzamido] isophtalimide:
   (a) On introduit 13 g du dérivé précédent, à 10°C, dans 10 ml d'une solution du chlorure de l'acide phtalimidoacétique préparé, avec 4,7 g d'acide phtaloylglycine, 1,7 ml de SOCl₂. Après 2 heures d'agitation à 20°C, la solution est versée dans 150 ml d'eau à 80°C et le précipité est isolé. p = 10,6 g.
   (b) Chlorure d'acide:
      On introduit 16 ml de chlorure de thionyle dans une solution de 12,4 g du dérivé précédent dans 60 ml de dioxanne et 10 ml de diméthylformamide, en maintenant la température à moins de 10°C. Après 30 minutes d'agitation, la solution est versée dans 200 ml d'eau et le précipité isolé est lavé avec 100 ml d'acétate d'éthyle. p = 12 g.
   (c) Condensation sur l'aminoalcool:
      A 60°C, on introduit 11,6 g du dichlorure d'acide dans 120 ml de N-méthylpyrrolidone contenant 13,5 g de 1-déoxy 1-(2,3-dihydroxypropyl-amino)D-galactitol préparé suivant G. Après 4 heures d'agitation, le mélange est introduit dans 1200 ml de dichlorométhane et le précipité isolé. p = 35 g.
   (d) Elimination du groupe protecteur phtalimide:
      14 g du dérivé précédent sont introduits dans 30 ml d'eau à 80°C, contenant 1,2 g d'hydrate d'hydrazine. Après 3 heures à cette température, la solution est acidifiée jusqu'à pH 1 par addition d'une solution aqueuse de HCl 5N. Le précipité formé après plusieurs heures est séparé et le filtrat traité successivement par les résines IMAC® HP 1110 Na et HP 661 (Rohm et Haas). HPLC: colonne No. 1; Eluant No. 2; tᵣ = 25-28 minutes (produit de départ 32-36 minutes).
3. Réaction avec le chelate A:
On introduit dans 15 ml d'eau, 0,6 g du chelate de gadolinium A (sel de Na), 0,7 g d'EDCI, HCI et 4 g de l'amine précédente; le pH est amené à 6 par addition d'une solution aqueuse de HCI 1N et le mélange maintenu 2 heures à 40°C, avant d'être introduit dans 200 ml d'éthanol. Le précipité formé est isolé et sa solution dans 100 ml d'eau soumise à une ultrafiltration sur une membrane de polyéthersulfone de seuil de coupure 5 KDa.
   HPLC: colonne et Eluant No. 3; tᵣ = 26 minutes.
   CES: Conditions No. 1; tᵣ = 36 minutes.

### Exemple 7.

Chelate de gadolinium de formule I dans laquelle m = 2
R' = 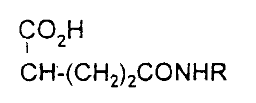 R = 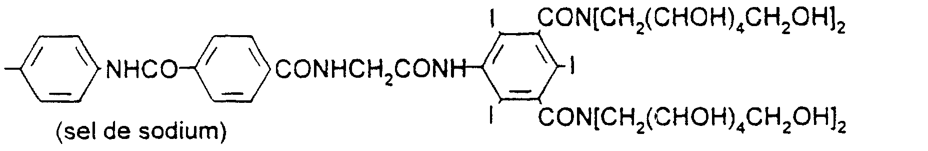
1. Acide 4-(4-aminophénylcarbamoyl)benzoïque:
   (a) 4-(4-nitrophénylcarbamoyl)benzoate de méthyle:
      Vers 10°C, on introduit peu à peu 10 ml de chlorure de thionyle dans une solution de 25 g de monoester méthylique de l'acide téréphtalique dans 125 ml de diméthylacétamide; après 1 heure 30 d'agitation à 15°C, le mélange est introduit lentement dans une solution de 19 g de 4-nitroaniline dans 125 ml du même solvant. Après 2 heures à 50°C, le précipité formé est isolé.
      Rendement: 97%.
   (b) Réduction:
      10 g du dérivé nitré précédent sont mis en suspension dans 100 ml de diméthylacétamide en présence de 2,5 g de Palladium sur charbon à 10% (humidité 50%) et maintenus sous une pression d'hydrogène de 0,5 Mpa pendant 7 heures. Le catalyseur est éliminé par filtration du milieu sur Clarcel® et le filtrat introduit dans 500 ml d'eau. Le précipité formé est isolé.
      Rendement: 97%.
   (c) Hydrolyse du groupe ester:
      9 g du composé précédent, 50 ml de NaOH aqueux (2N) et 25 ml de méthanol sont maintenus à 60°C pendant 2 heures. La solution est acidifiée à 20°C jusqu'à pH 4,5 par addition d'une solution d'acide chlorhydrique concentrée (37%) et 7 g du précipité formé sont isolés.
      HPLC: Colonne No. 1; Eluant No. 2; tᵣ = 29 minutes.
2. N,N'-bis(2,3,4,5,6-pentahydroxyhexyl) 5-[4-(4-aminophényl carbamoyl)benzoylglycylamino] 2,4,6-triiodo isophtalamide:
   On agite 2 heures à température ambiante, 25 g de l'amino acétamido isophtalamide iodée préparée en F avec 3 ml de triéthylamine, 5,8 g du produit précédent, 4,3 g de HOBT et 6 g d'EDCI, HCI, puis on le verse sur 700 ml de dichlorométhane. Le précipité formé est isolé et purifié par chromatographie sur une colonne de silice silanisée RP-2 (Merck), en éluant avec de l'eau. On isole 16 g du produit cherché.
   HPLC: Colonne No. 1; Eluant No. 2; t, = 14 minutes.
3. Couplage avec le chelate de gadolinium A:
   On maintient quelques jours sous agitation, à 40°C, 0,125 g du chelate (sel de Na), 1,8 g du composé précédent, 0,15 g d'EDCI, HCl et 100 mg de NHS sulfonique en maintenant le pH vers 7, par addition d'une solution aqueuse de HCl 1N. Le précipité formé par introduction de 50 ml d'éthanol est isolé et purifié par ultrafiltration sur membrane de 5 kDa pour donner 1 g du produit attendu.
   CES: Conditions No. 1; tᵣ = 30 minutes.
   HPLC: Colonne No. 3; Eluant No. 3; tᵣ = 20 minutes.

### Exemple 8.

Chelate de gadolinium de formule I dans laquelle m = 2
R' = 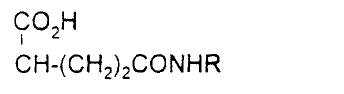 R = 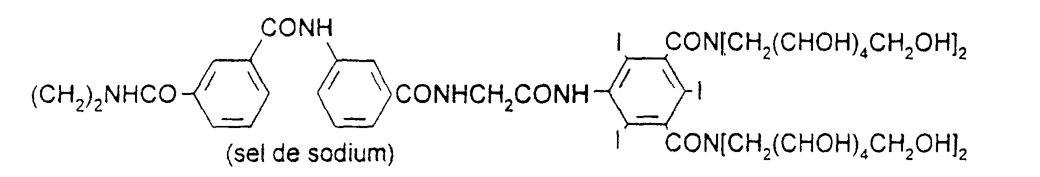
1. Acide 3-[3-(phtalimidoéthylcarbamoyl)benzamido]benzoïque:
   (a) Acide 3-(aminoéthylcarbamoyl)benzoïque:
      Une solution de 9 g de mono ester méthylique d'acide isophtalique et 1,5 ml d'éthylènediamine dans 90 ml de méthanol est agitée pendant 18 heures avant concentration à sec. Le résidu est lavé avec du méthanol glacé.
      Rendement: 91%.
   (b) Protection du NH₂ sous forme de phtalimide:
      On introduit 9 g d'amine et 14 g de N-carbéthoxyphtalimide dans une solution de 6,9 g de carbonate de sodium dans 180 ml d'eau. Après 1 heure 30 d'agitation, le solide est isolé et le filtrat acidifié jusqu'à pH 2. Le phtalimide précipite sous forme de cristaux blancs.
      Rendement: 80%.
      HPLC: Colonne No. 1; Eluant No. 2; tᵣ = 32 minutes.
   (c) On ajoute à 10°C goutte à goutte, 2,6 ml de chlorure de thionyle à une solution de 12 g du phtalimide précédent dans 45 ml de diméthylacétamide. Après 2 heures à 15°C, la solution orangée est ajoutée lentement à une solution de 5 g d'acide 3-aminobenzoïque dans 45 ml de diméthylacétamide. Le milieu réactionnel est maintenu 3 heures à 55°C puis versé dans 900 ml d'eau. Le précipité formé est isolé.
      Rendement: 96%.
2. N,N'-bis(2,3,4,5,6-pentahydroxyhexyl) 5-[3-[3-(aminoéthylcarbamoyl)benzamido]benzamidoacétamido] 2,4,6-triiodoisophtalamide:
   (a) 20 g de l'aminoacétamidoisophtalamide iodée préparé selon F, 2,5 ml de triéthylamine, 8,3 g du composé précédent, 3,4 g d'HOBT et 4,9 g d'EDCI, HCI dans 100 ml de diméthylacétamide sont maintenus 1 heure 30 sous agitation. Le milieu réactionnel est alors versé dans 700 mi de dichlorométhane et 26 g de précipité formé sont isolés.
      HPLC: Colonne No. 1; Eluant No. 2; tᵣ = 26 minutes.
   (b) Hydrazinolyse:
      On introduit 3,5 ml d'hydrate d'hydrazine dans une solution de 25 g du produit précédent dans 55 ml d'eau à 80°C. Après 8 heures à cette température, le milieu est acidifié jusqu'à pH 1 et le précipité formé est isolé. Il est purifié par chromatographie sur une résine cationique (H⁺) puis anionique (OH⁻). Rendement 70%.
      HPLC: Colonne No. 1; Eluant No. 2; tᵣ = 23 minutes.
3. Couplage avec le chelate de gadolinium A:
   On maintient à pH 7 pendant 1 heure à 40°C sous agitation 2 g du chelate A (sel de Na), 2,3 g d'EDCI, HCI et 16 g du composé précédent dans 50 ml d'eau, avant d'ajouter 200 mg de NHS sulfonique et de poursuivre l'agitation à 40°C pendant 3 heures. Le milieu est alors versé dans 500 ml d'éthanol et le précipité isolé est purifié par ultrafiltration pour donner 10 g du sel de sodium du produit cherché.
   CES: Conditions No. 1; tᵣ = 36 minutes.
   HPLC: Colonne No. 3; Eluant No. 3, tᵣ = 21 minutes.

### Exemple 9.

Chelate de gadolinium de formule I dans laquelle m = 2
R = 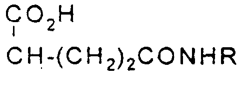 R = 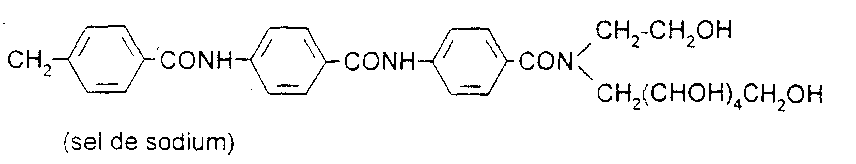
1. Acide 4-[4-(4-[phtalimidométhyl]benzamido)benzamido]benzoïque:
   A 15°C, on introduit 3,5 ml de chlorure de thionyle dans une solution de 19,3 g d'acide 4-[4-[phtalimidométhyl]benzamido]benzoïque, puis après 2 heures 45 d'agitation, 8 g d'acide 4-aminobenzoïque. Après 12 heures à 20°C, le milieu est versé dans 500 ml d'eau. Le précipité formé est lavé au dioxanne. p = 11,5 g.
   HPLC: colonne No. 1; Eluant No. 2; tᵣ = 53 minutes.
2. N,N'-(2,3,4,5,6-pentahydroxyhexyl) N,N'-(2-hydroxyéthyl) 4-[4-[4-(aminométhyl)benzamido]benzamido]benzamide:
   (a) On maintient à 20°C, une nuit sous agitation, 4,8 g de 1-deoxy 1-(2-hydroxyéthylamino)D-glucitol, 4 g de HOBT, H₂O, 11 g de l'acide précédent et 6,7 g de EDCI, HCI. Le milieu réactionnel est alors versé dans 500 ml de dichlorométhane et le précipité formé est lavé par 300 ml d'éther diéthylique. HPLC: colonne No. 1; Eluant No. 2; tᵣ = 39 minutes.
   (b) Hydrazinolyse:

   On introduit le précipité précédent dans 60 ml de diméthylacétamide et à 80°C, 3,2 ml d'hydrate d'hydrazine en solution dans 25 ml d'eau. Après 3 heures le milieu est concentré et le résidu dissous dans de l'eau. La phase aqueuse est amenée à pH 7,5 par addition d'une solution concentrée de HCl, puis chromatographiée sur une résine cationique forte (H⁺) puis anionique faible (OH⁻). Après concrétisation dans l'éthanol, on obtient 3,5 g de précipité.
   HPLC: Colonne No. 1; Eluant No. 2; tᵣ = 27 minutes.
3. Couplage avec le chelate de gadolinium A:
   On porte à 40°C 1,5 g du composé précédent avec 0,5 g du chelate A dans 15 ml d'eau et 5 ml de diméthylacétamide et on introduit 0,75 g d'EDCI, HCI et 0,05 g de NHS sulfonique (sel de sodium). Après 2 heures d'agitation à cette température, le milieu réactionnel est versé sur 2 volumes d'éthanol et 1,3 de précipité sont isolés.
   HPLC: Colonne No. 3; Eluant No. 3; tᵣ = 31 minutes.

### Exemple 10.

Chelate de gadolinium de formule I dans laquelle m = 2
R' = 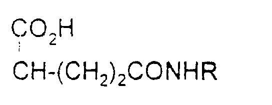 R = 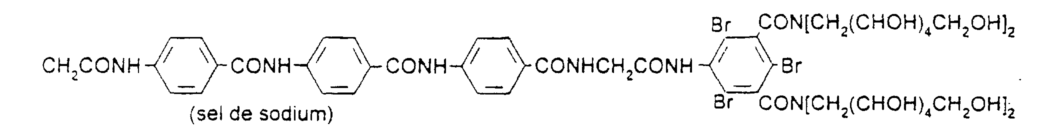
1. Acide 4-[4-[4-nitrobenzamido]benzamido] benzoïque:
   On introduit dans une solution à 5°C, 35 g d'acide 4-(4-aminobenzamido)benzoïque dans 140 ml de diméthylacétamide, 25,4 g de chlorure d'acide 4-nitrobenzoïque. Après une journée d'agitation à 20°C, le milieu réactionnel est versé sur 500 ml de dichlorométhane. p = 50 g.
2. Réduction du groupe nitro:
   Une suspension de 13 g de l'acide précédent dans 400 ml de diméthylacétamide est hydrogéné sous pression de 0,15 MPa, pendant 4 heures en présence de 3 g de Pd/C à 5% et 50% d'eau. Après filtration et évaporation du solvant, on isole 12 g de produit.
3. Acide 4-[4-[4-(phtalimidoacétamido)benzamido]benzamido] benzoïque:
   Une solution de 16,4 g d'acide phtalimidoacétique dans 40 ml de chlorure de thionyle est maintenue 4 heures à sa température de reflux. Après concentration, le milieu réactionnel est introduit dans 100 ml d'éther de diisopropyle. On isole 10 g de précipité de chlorure d'acide. 8,5 g de celui-ci sont introduits dans une solution de 12 g de l'aniline, obtenue à l'étape 2, en solution dans 50 ml de diméthylacétamide à 10°C. Après retour à 20°C, le solvant est éliminé par distillation et le résidu lavé par l'éther diéthylique.
   p = 16 g.
4. Condensation sur l'amine préparée en E et hydrazinolyze:
   18 g de l'amine E, 10 g de l'acide précédent et 3,5 g d'HOBT sont mis en solution dans 80 ml de diméthylacétamide à 35°C; on introduit ensuite 4,9 g d'EDCI, HCI dans le milieu réactionnel refroidi à 20°C; après une journée, celui-ci est versée sur 600 ml de dichlorométhane et le précipité formé est lavé avec 400 ml d'éthanol. Le précipité est dissous dans 90 ml de diméthylacétamide et 22 ml d'eau à 80°C; 1,85 ml d'hydrate d'hydrazine sont alors introduits et le milieu est agité pendant 3 heures avant d'évaporer les solvants. Le résidu est dissous dans 800 ml d'eau et le pH amené à 1 par addition d'une solution aqueuse de HCI (12N). Après filtration sur Celite® et chromatographie sur une résine anionique et sur une résine cationique, puis filtration de l'éluant sur noir de carbone, on isole 13 g du composé cherché.
5. Réaction des produits de formule II et III:
   1 g du chelate A et 8 g de l'amine ci-dessus sont dissous dans 40 ml d'eau. Après acidification jusqu'à pH 6 par addition d'une solution aqueuse diluée de HCI, on porte le milieu réactionnel à 40°C et on y introduit 0,09 g de NHS sulfonique et 1,2 g d'EDCI, HCI. Après 2 heures d'agitation à cette température, le milieu est versé dans 400 ml d'éthanol et le précipité isolé.
   Après les traitements de purification habituels, on isole 6 g du produit final.
   CES: Conditions précédentes; tᵣ = 38 minutes.

### Exemple 11.

Chelate de gadolinium de formule I dans laquelle m = 2
R' = 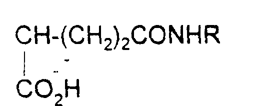 R = 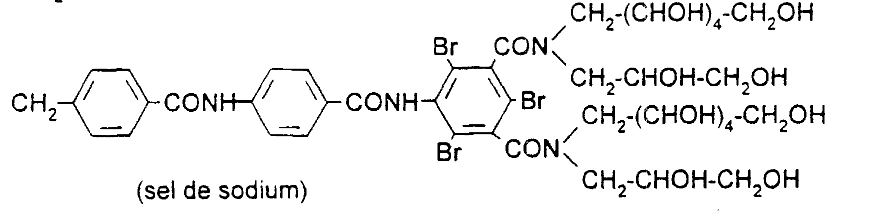
1. Acide 5-(4-nitrobenzamido) 2,4,6-tribromo isophtalique:
   On maintient pendant 18 heures à sa température de reflux, 50 g de chlorure d'acide para-nitrobenzoïque et 75 g d'acide 5-amino 2,4,6-tribromo isophtalique dans 400 ml de dioxanne. Après refroidissement, le précipité est filtré, lavé par 50 ml de dioxanne et séché. p = 115 g.
2. Acide 5-(4-aminobenzamido) 2,4,6-tribromo isophtalique:
   Une solution de 180 g du dérivé nitré précédent dans 600 ml d'eau est amenée à pH 6 par addition d'une solution aqueuse de NaOH 5N et hydrogénée sous une pression de 5x10⁵ Pa en présence de Pt type 156 (Johnson Matthey) pendant 7 heures. Le catalyseur est séparé par filtration et l'eau évaporée sous pression réduite. p = 80 g.
   HPLC: Colonne No. 1; Eluant No. 6: eau/acide trifluoroacétique (pH 2,8) avec méthanol (99/1 - V/V);
   débit 1 ml/min.; tᵣ = 3,6 minutes (18,8 minutes pour le nitré).
3. Acide 5-(4-[4-(phtalimidométhyl)benzamido]benzamido) 2,4,6-tribromo isophtalique:
   Un mélange de 10 g d'acide 4-aminométhylbenzoïque, 14,5 g de N-carbéthoxyphtalimide et 9,2 ml de triéthylamine dans 140 ml de tétra-hydrofuranne est maintenu à sa température de reflux pendant 72 heures. Le précipité isolé par filtration à température ambiante du milieu réactionnel est lavé avec du diéthyléther et une solution aqueuse d'acide chlorhydrique 1N. On obtient 14,5 g de solide, dont 12,2 g sont dissous à 10°C dans 90 ml de N,N-diméthylacétamide et 3,5 ml de chlorure de thionyle; après 3 heures d'agitation, on introduit dans le milieu 23,4 g de l'aniline obtenue à l'étape 2 et on laisse sous agitation pendant 1 nuit avant de verser le mélange dans 900 ml d'eau. Le précipité isolé, lavé à l'eau est recristallisé dans 200 ml de dioxanne. p = 30 g.
   HPLC: Colonne No. 1; Eluant No. 5: eau/CH₃COONH₄ (0,01 M) / CH₃CN;
   débit 1 ml/min.; gradient de 85/15 à 50/50 en 20 minutes.
4. Dichlorure d'acide:
   30,3 g du dérivé isophtalique obtenu à l'étape précédente sont dissous dans 150 ml de dioxanne contenant 26 ml de diméthylformamide et à 5°C, 42 ml de chlorure de thionyle sont introduits goutte à goutte. Après 30 minutes à O°C, le mélange est versé dans 550 ml d'eau et le précipité formé est filtré, lavé à l'eau et à l'éther diisopropylique. p = 26 g après séchage.
5. N,N'-(2,3,4,5,6-pentahydroxyhexyl)N,N'-(2,3-dihydroxypropyl) 2,4,6-tribromo 5-[4-(4-aminométhylbenzamido)benzamido] isophtalamide:
   (a) 10 du dichlorure d'acide sont introduits dans une solution de 15 g de 1-déoxy-1-(2,3-dihydroxypropylamino)-D-galactitol dans 100 ml de N-méthylpyrrolidone à 60°C. Après 4 heures d'agitation à cette température, le milieu, revenu à température ambiante, est versé dans 1 litre d'isopropanol. Le précipité formé est isolé et séché.
      HPLC: Colonne No. 1; Eluant No. 5; tᵣ = 16 minutes.
   (b) Elimination du groupe phtalimide:
      20,4 g du solide précédent sont introduits sous agitation dans 80 ml de N,N-diméthylacétamide à 80°C suivis par 1,6 ml d'hydrate d'hydrazine en solution dans 20 ml d'eau. Après 3 heures à cette température, le milieu réactionnel est versé à température ambiante dans 1 litre d'éthanol. Le précipité formé est isolé, séché puis dissous dans 40 ml d'eau. A 0°C on introduit environ 2 ml de solution aqueuse de HCI 6N pour abaisser le pH à 2; le milieu est filtré sur Célite® puis purifié par passage sur des résines échangeuses d'ions (Amberlite® anionique et IMAC® cationique). On obtient alors 6 g du produit cherché.
      HPLC: Colonne No. 1; Eluant No. 5; tᵣ = 24 à 29 minutes.
6. Réaction avec le chelate A:
   5,8 g de l'amine précédente et 1,1 g du chelate (sel de sodium) sont dissous dans 12,5 ml d'eau et le pH est descendu à 6 par addition d'une solution aqueuse de HCI 1N. On introduit alors 12,5 ml de dioxanne, 0,06 g de HOBT puis 1,8 g d'EDCI et on maintient le milieu à pH 6 pendant 4 heures avant de le verser dans 150 ml d'éthanol. Le précipité formé est isolé puis purifié par ultrafiltration tangentielle sur une membrane de seuil de coupure 30 KD en cellulose régénérée de 50 cm² (module Labscale® de Millipore®). On isole ainsi 5,6 g du produit cherché.
   HPLC: Colonne No. 1; Eluant No. 3; tᵣ = 16 minutes.

### Exemple 12.

Chelate de gadolinium de formule I dans laquelle m = 2,
R' = 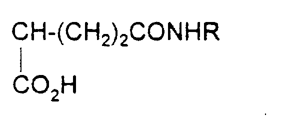 R = 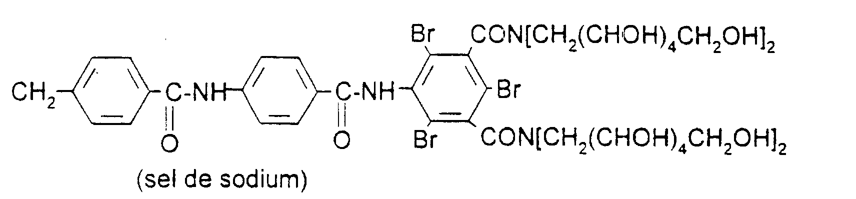
1. N,N'-bis(2,3,4,5,6-pentahydroxyhexyl) 2,4,6-tribromo 5-[4-(4-amino-méthylbenzamido) benzamide] isophtalamide:
   (a) On agite pendant 4 heures à 60°C, une solution dans 200 ml de N-méthylpyrrolidone de 10 g du dichlorure d'acide préparé comme dans l'étape 4 de l'exemple 11 et 20,6 g de disorbitylamine. Le milieu réactionnel est ensuite introduit dans 1,5 litre d'isopropanol d'où on isole 17,5 g de précipité.
      HPLC: Colonne No. 1; Eluant No. 5; tᵣ = 15 minutes.
   (b) Elimination du groupe phtalimide:
      On opère comme dans l'exemple précédent à partir de 16 g du produit de l'étape (a) pour obtenir 6,2 g de l'amine cherchée.
      HPLC: Colonne No. 1; Eluant No. 2; tᵣ = 15 à 20 minutes.
2. On opère comme dans l'exemple 11 précédent à partir de 0,78 g de chelate A et 4,8 g d'amine pour obtenir 2,5 g du produit cherché.
   HPLC: Colonne No. 1; Eluant No. 3; tᵣ= 13 minutes.

### Exemple 13.

Chelate de gadolinium de formule I dans laquelle m = 2
R' = 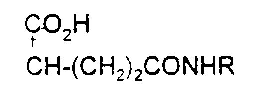 R = 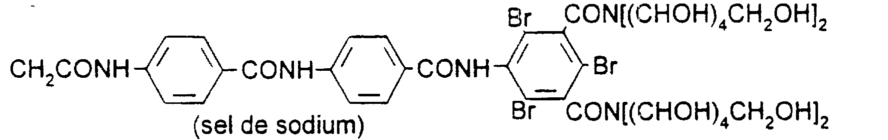
1. Acide 4-4-(phtalimidométhylcarbonylamino) benzoïque:
   On introduit à 10°C, 3,9 ml de chlorure de thionyle dans une solution de 10 g de phtaloylglycine dans 30 ml de N,N-diméthylacétamide puis après 3 heures d'agitation à cette température, 4 g d'acide 4-aminobenzoïque. Après une nuit à température ambiante, le milieu est versé dans 500 ml d'eau à 80°C. Le précipité formé est isolé.
2. Acide 5-(4-[4-(phtalimidométhylcarbonylamino)benzamido] benzamido) 2,4,6-tribromo isophtalique:
   On introduit à 10°C, 4,8 ml de chlorure de thionyle dans une solution de 19,5 g de l'acide précédent dans 120 ml de N,N-diméthylacétamide, puis, après 2 heures 30, 38 g d'acide 5-(4-amino)benzamido 2,4,6-tribromo isophtalique. Après une nuit à température ambiante, le milieu est versé sur 1 litre d'eau et le précipité formé est isolé et lavé avec 400 ml de dioxanne à chaud. p = 7,4 g.
3. N,N'-bis(2,3,4,5,6-pentahydroxyhexyl) 2,4,6-tribromo 5-(4-(4-[phtalimidométhylcarbonylamino]benzamido)benzamido) isophtalamide:
   On introduit à 5°C, 21 ml de chlorure de thionyle dans une solution de 16,5 g du diacide précédent dans 90 ml de dioxanne et 13,6 ml de diméthyl formamide. Après 2 heures 30 d'agitation, le milieu est versé sur 400 ml d'eau et le précipité formé est séché puis introduit dans une solution à 70°C de 29,3 g de disorbitylamine dans 150 ml de N-méthylpyrrolidone. Après 4 heures d'agitation à cette température, le milieu est ramené à température ambiante, les sels sont isolés et le filtrat versé dans 1 litre d'isopropanol. Le précipité formé est lavé avec 1,2 litre d'éthanol. p = 23 g.
   HPLC: Colonne No. 1; Eluant No. 2; tᵣ= 28 à 33 minutes.
4. Elimination du groupe phtalimide:
   On introduit dans un mélange de 80 ml de N,N-diméthylacétamide et 20 ml d'eau à 80°C, 22 g du phtalimide précédent et 1,4 ml d'hydrate d'hydrazine. Après 3 heures à cette température, le milieu est ramené vers 20°C puis versé sur 300 ml d'éthanol. Le précipité formé, séché, est dissous dans 40 ml d'eau, la solution refroidie à 0°C et 3 ml d'acide chlorhydrique 6N sont ajoutés jusqu'à pH 1,5. Après filtration sur Celite®, le filtrat est élué sur 45 ml de résine anionique Amberlite® IRA67 (Rohm et Hass), 50 ml de résine cationique IMAC® HPIIIE puis 16 ml de résine anionique Amberlite® IRA458. Après neutralisation et filtration sur une membrane de 0,22 µm, la solution est amenée à sec. p = 11,7 g.
   HPLC: Colonne No. 1; Eluant No. 2; tᵣ = 16 à 20 minutes.
5. Condensation sur le chelate A:

11,2 g de l'amine précédente et 1,9 g du chelate A (sel de sodium) sont mis en solution dans 24 ml d'eau. Le pH de la solution est amené à 6,1 par addition d'une solution aqueuse de HCI 2N. On introduit alors 24 ml de dioxanne puis 2,7 g de chlorhydrate de 1-(3-diméthylaminopropyl)-3-éthylcarbodiimide et 0,094 g d'hydroxy benzotriazole. Après 3 heures d'agitation à température ambiante, le mélange est versé sur 300 ml d'éthanol et le précipité formé isolé. Après ultrafiltration avec un module Labscale® dont la membrane a un seuil de coupure de 10 KD, on lyophilise la solution. p = 10,2 g.
HPLC; Colonne No. 1; Eluant No. 7: CH₃CN/P.I.C. A* (Waters®) gradient en 25 minutes de 25/75 à 30/70; P.I.C. A = H₂O/H₃PO₄/(C₄H₉)₄N⁺HSO₄
tᵣ = 14 minutes.

### Exemple 14

Chelate de gadolinium de formule I dans laquelle m= 1
R' = 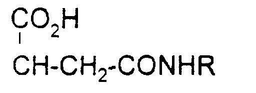 R =

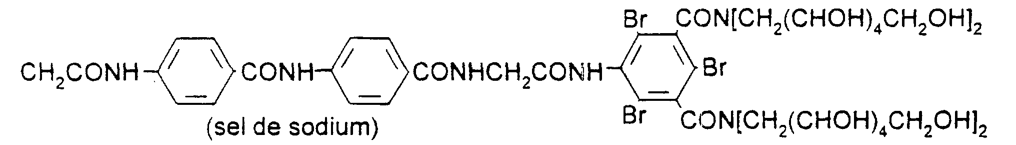

On dissout 1,8 g de chelate de gadolinium de l'acide (1,4,7,10-tétraazacyclododécane) 1,4,7,10-tétra(2-succinique) et 13,5 g de N,N'-bis(2,3,4,5,6-pentahydroxyhexyl) 2,4,6-tribromo 5-(4-[4-aminoacétamidobenzamido] benzoylglycylamino) isophtalamide préparé comme à l'étape f de l'exemple 3 dans 70 ml d'eau et on introduit une solution aqueuse d'HCI 1N jusqu'à pH 6. Après addition de 2 g d'EDCI le mélange est maintenu 2 heures à 40°C. On effectue alors une ultrafiltration tangentielle sur une cassette Minisette® Filtron® comportant une membrane de polyéthersulfone de seuil de coupure 5 KD, jusqu'à 2 litres de filtrat; le rétentat est ensuite mis en contact pendant 2 heures avec 15 g de noir Darco® G60 commercialisé par Aldrich®. Après séparation du noir par filtration et concentration à sec, on isole 5 g de solide blanc.
CES: Conditions No. 1; tᵣ = 35 minutes.

## Revendications

1. Chelate d'un cation d'un métal paramagnétique avec un composé de formule 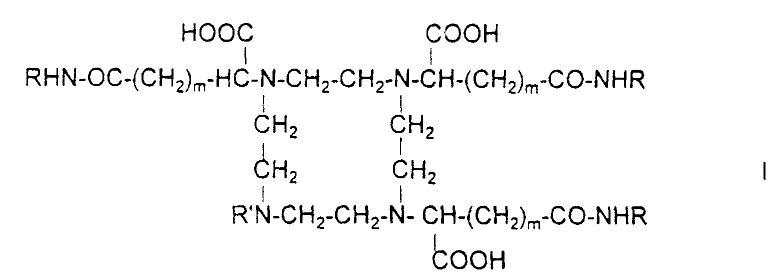 dans laquelle
m est 1 ou 2
R' est H, un groupe (C₁-C₄) alkyl ou hydroxyalkyl, un groupe CH₂-COOH ou CH₂-CONZ₁Z₂, Z₁ et Z₂ étant indépendamment l'un de l'autre, H, ou un groupe (C₁-C₄) alkyl, éventuellement hydroxylé,
ou R' est un groupe 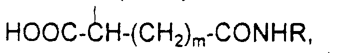 R est un groupe 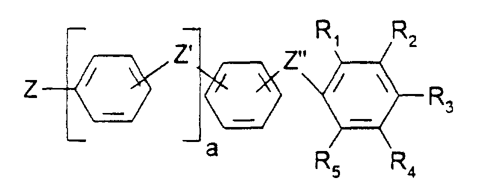 dans lequel a est 1 ou 2
Z est une liaison, CH₂, CH₂CONH ou (CH₂)₂NHCO
Z' est une liaison, O, S, NQ, CH₂, CO, CO-NQ, NQ-CO, NQ-CO-NQ ou CO-NQ-CH₂-CONQ
Z" est CO-NQ, NQ-CO ou CO-NQ-CH₂-CO-NQ, NQ-CO-CH₂-NQ-CO
avec Q est H ou un groupe (C₁-C₄) alkyl, éventuellement hydroxylé
R₁, R₂, R₃, R₄, R₅, indépendamment l'un de l'autre, sont choisis parmi H, Br, Cl, I, CO-NQ₁Q₂ ou N(Q₁)-CO-Q₂ et Q₁ et Q₂, identiques ou différents, sont choisis parmi les groupes (C₂-C₆) alkyl, éventuellement hydroxylés, éventuellement interrompus par un atome d'oxygène, de telle sorte que Q₁ et Q₂ comportent à eux deux de 4 à 10 groupes OH,
étant entendu qu'au moins 1, et au plus 2 groupes R₁, R₂, R₃, R₄, R₅, sont des groupes amides,
et ses sels avec une base minérale, ou une base organique, pharmacologiquement acceptable.

2. Chelate d'un cation d'un métal paramagnétique avec un composé de formule I, et ses sels, selon la revendication 1 dans laquelle
m est 2
R' est 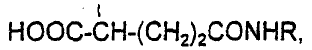 CH₂COOH, ou CH₂CONZ₁Z₂,
a est 1,
Z est une liaison, CH₂, ou CH₂CONH,
Z' est CONH, NHCO, CONH-CH₂-CONH, ou NHCONH,
Z" est CONH ou CONH-CH₂-CONH, et alors R₂=R₄=CONQ₁Q₂ et R₁, R₃, R₅ sont
H, Br, Cl ou I ou R₃ = CONQ₁Q₂, et R₁, R₂, R₄, R₅ sont H, Br, Cl ou I,
ou Z" est NHCO, et alors R₂ = R₄ = N(Q₁)COQ₂ ou R₃ = N(Q₁)-COQ₂, et R₁, R₂, R₄, R₅ sont H, Br, Cl ou I.

3. Chelate d'un cation métallique paramagnétique d'un composé de formule I, et ses sels, selon la revendication 1 dans laquelle
m est 2
R' est 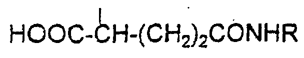 ou CH₂COOH,
a est 1,
Z est une liaison, CH₂ ou CH₂CONH,
Z' est CONH, NHCONH, ou CONH-CH₂-CONH,
Z" est CONH, ou CONH-CH₂-CONH,
R₁, R₃ et R₅ sont tous les trois Br ou I,
R₂ et R₄ sont CONQ₁Q₂ avec Q₁ et Q₂ choisis parmi les groupes (C₂-C₆) alkyl hydroxylés comportant à eux deux 6 à 10 groupes OH, ou comportant 4 à 8 groupes OH si Q₁ et/ou Q₂ est interrompu par un atome d'oxygène.

4. Chelate d'un cation métallique paramagnétique d'un composé de formule I, et ses sels, selon la revendication 1 dans laquelle
m est 2
R' est 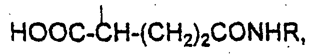 a est 1,
Z est CH₂ ou CH₂CONH,
Z' est CONH, NHCONH,
Z" est CONH, ou CONH-CH₂-CONH,
R₁, R₃ et R₅ sont tous les trois Br ou I,
R₂ et R₄ sont CONQ₁Q₂ avec Q₁ et Q₂ choisis parmi les groupes (C₂-C₆) alkyl hydroxylés comportant à eux deux 6 à 10 groupes OH, ou comportant 4 à 8 groupes OH si Q₁ et/ou Q₂ est interrompu par un atome d'oxygène.

5. Chelate d'un cation métallique paramagnétique d'un composé de formule I, et ses sels, selon la revendication 1 dans laquelle
m est 2
R' est CH₂CO₂H, ou 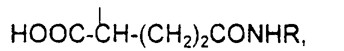 a est 1,
Z est CH₂, ou CH₂CONH,
Z' et Z" sont CONH,
R₁, R₃ et R₅ sont tous les trois Br ou I,
R₂ et R₄ sont CONQ₁Q₂ avec Q₁ et Q₂ choisis parmi les groupes (C₂-C₆) alkyl hydroxylés comportant à eux deux 6 à 10 groupes OH, ou comportant 4 à 8 groupes OH si Q₁ et/ou Q₂ est interrompu par un atome d'oxygène.

6. Chelate d'un cation métallique paramagnétique d'un composé de formule I, et ses sels, selon la revendication 1 dans laquelle
m est 2
R' est CH₂CO₂H, ou 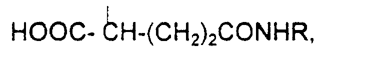 a est 2,
Z est CH₂ ou CH₂CONH,
Z' est CONH, ou NHCO,
Z" est CONH, ou CONH-CH₂-CONH,
et R₁, R₃ et R₅ sont tous les trois Br ou I,
et alors R₂ et R₄ sont tous les deux CONQ₁Q₂,
ou R₃ est CONQ₁Q₂ et alors R₁, R₂, R₄ et R₅ sont H, Br, ou I.

7. Chelate d'un cation métallique paramagnétique d'un composé de formule I, et ses sels, selon la revendication 1 dans laquelle
m est 1
R' est 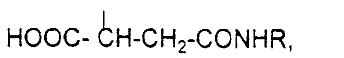 a est 1,
Z est CH₂, ou CH₂CONH,
Z' est CONH,
Z" est CONH, ou CONH-CH₂-CONH,
R₁, R₃ et R₅ sont Br,
R₂ et R₄ sont CONQ₁Q₂ avec Q₁ et Q₂ sont des groupes (C₂-C₆)alkyl hydroxylés comportant à eux deux de 6 à 10 groupes OH.

8. Chelate selon l'une des revendications 1 à 7 dans lequel le cation métallique est le Gd³⁺.

9. Chelate de gadolinium, et ses sels, du composé de formule I selon la revendication 1 dans laquelle
m est 2
R' = 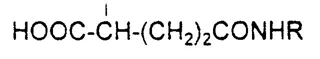 et R est

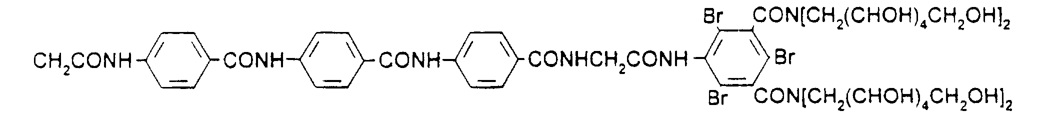

10. Chelate de gadolinium, et ses sels, du composé de formule I selon la revendication 1 dans laquelle
m est 2
R' est 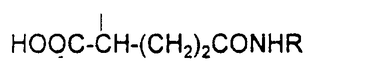 R = 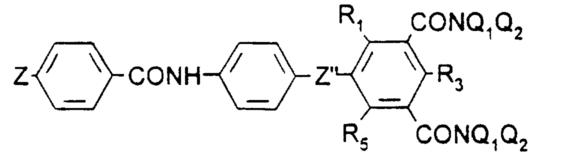
- avec Z" est CONH-CH₂CONH et Q₁ et Q₂ sont CH₂(CHOH)₄CH₂OH, alors, soit Z est CH₂ et R₁, R₃ et R₅ sont I, soit Z est CH₂CONH et R₁, R₃ et R₅ sont Br,
- ou avec Z" est CONH, Z est CH₂ ou CH₂CONH, et R₁, R₃, R₅ sont Br et Q₁ est CH₂(CHOH)₄CH₂OH, et Q₂ est CH₂CHOH-CH₂OH, ou CH₂(CHOH)₄CH₂OH.

11. Produit de contraste pour imagerie diagnostique par résonance magnétique comprenant une quantité efficace d'un chelate selon l'une des revendications 1 à 10 dans un véhicule pharmaceutiquement acceptable.

12. Produit de scintigraphie comportant un chelate radiomarqué d'un cation métallique avec un composé de formule I tel que défini à la revendication 1.

## Claims

1. Chelate of a cation of a paramagnetic metal with a compound of formula 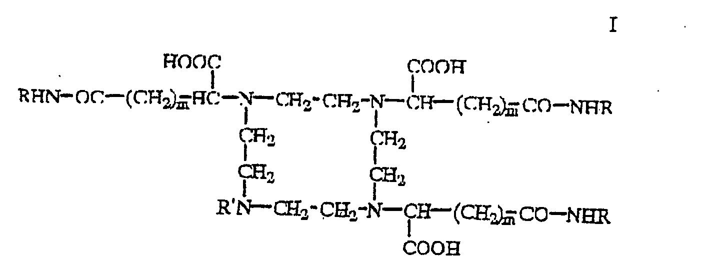 in which
m is 1 or 2,
R' is selected from the group consisting of H, a C₁-C₄ alkyl or hydroxyalkyl group, a group CH₂-COOH and a group CH₂-CONZ₁Z₂, Z₁ and Z₂ being, independently of each other, H or an optionally hydroxylated C₁-C₄ alkyl group,
or R' is a group 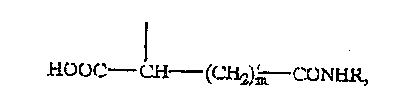 R is a group 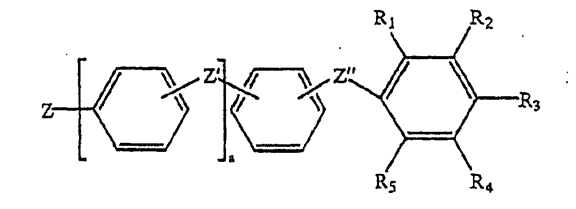 in which a is 1 or 2
Z is selected from the group consisting of a bond, CH₂, CH₂CONH and (CH₂)₂NHCO Z' is selected from the group consisting of a bond, O, S, NQ, CH₂, CO, CO-NQ, NQ-CO, NQ-CO-NQ and CO-NQ-CH₂-CONQ
Z" is selected from the group consisting of CO-NQ, NQ-CO, CO-NQ-CH₂-CO-NQ and NQ-CO-CH₂-NQ-CO
where Q is H or an optionally hydroxylated C₁-C₄ alkyl group,
R₁, R₂, R₃, R₄ and R₅, independently of each other, are selected from the group consisting of H, Br, Cl, I, CO-NQ₁Q₂ and N(Q₁)-CO-Q₂ and Q₁ and Q₂ are independently selected from optionally hydroxylated C₂-C₆ alkyl groups which are optionally interrupted by an oxygen atom, such that Q₁ and Q₂ together contain from 4 to 10 OH groups, with the proviso that at least 1, and not more than 2, groups R₁, R₂, R₃, R₄ and R₅ are amido groups,
and the salts thereof with a pharmacologically acceptable organic base or inorganic base.

2. Chelates of a cation of a paramagnetic metal with a compound of formula I, and the salts thereof, according to claim 1, in which
m is 2
R' is 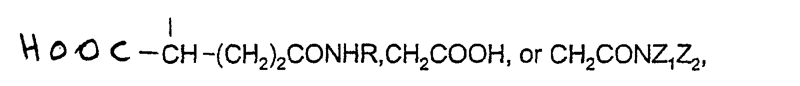 a is 1,
Z is selected from the group consisting of a bond, CH₂ and CH₂CONH,
Z' is selected from the group consisting of CONH, NHCO, CONH-CH₂-CONH and NHCONH,
Z" is selected from the group consisting of CONH and CONH-CH₂-CONH, and then R₂=R₄=CONQ₁Q₂ and R₁, R₃, and R₅ are H, Br, Cl or I, or R₃ = CONQ₁Q₂ and R₁, R₂, R₄, R₅ are H, Br, Cl or I.
or Z" is NHCO, and then R₂=R₄=N(Q)₁COQ₂ or R₃=N(Q₁)-COQ₂ and R₁, R₂, R₄ and R₅ are H, Br, Cl or I.

3. Chelate of a paramagnetic metal cation of a compound of formula I, and the salts thereof, according to claim 1, in which
m is 2,
R' is 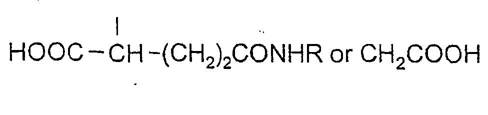 a is 1,
Z is selected from the group consisting of a bond, CH₂ and CH₂ CONH,
Z' is selected from the group consisting of CONH, NHCONH and CONH-CH₂-CONH,
Z" is selected from the group corisisting of CONH and CONH-CH₂-CONH,
R₁, R₃ and R₅ are all Br or I,
R₂ and R₄ are CONQ₁Q₂ where Q₁ and Q₂ are selected from C₂-C₆ hydroxyalkyl groups together containing 6 to 10 OH groups or containing 4 to 8 OH groups if Q₁ and/or Q₂ is interrupted by an oxygen atom.

4. Chelate of a paramagnetic metal cation of a compound of formula I, and the salts thereof, according to claim 1, in which
m is 2,
R is 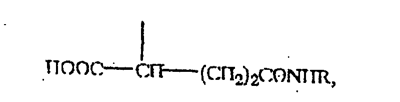 a is 1,
Z is selected from the group consisting of CH₂ and CH₂CONH,
Z' is selected from the group consisting of CONH and NHCONH,
Z" is selected from the group consisting of CONH and CONH-CH₂-CONH,
R₁, R₃ and R₅ are all Br or I,
R₂ and R₄ are CONQ₁Q₂ where Q₁ and Q₂ are selected from hydroxylated C₂-C₆ alkyl groups together containing 6 to 10 OH groups or containing 4 to 8 OH groups if Q₁ and/or Q₂ is interrupted by an oxygen atom.

5. Chelate of a paramagnetic metal cation of a compound of formula I, and the salts thereof, according to claim 1, in which
m is 2,
R is
CH₂CO₂H, or 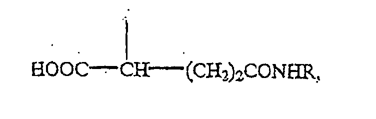 a is 1,
Z is selected from the group consisting of CH₂ and CH₂CONH,
Z' and Z" are CONH₂,
R₁ R₃ and R₅ are all Br or I,
R₂ and R₄ are CONQ₁Q₂ where Q₁ and Q₂ are selected from hydroxylated C₂-C₆ alkyl groups together containing 6 to 10 OH groups or containing 4 to 8 OH groups if Q₁ and/or Q₂ is interrupted by an oxygen atom.

6. Chelate of a paramagnetic metal cation of a compound of formula I, and the salts thereof, according to claim 1, in which
m is 2,
R' is
CH₂CO₂H, or 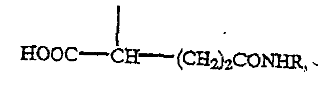 a is 2,
Z is selected from the group consisting of CH₂ and CH₂CONH,
Z' is selected from the group consisting of CONH and NHCO,
Z" is selected from the group consisting of CONH and CONH-CH₂-CONH,
and R₁, R₃ and R₅ are all Br or I,
and then R₂ and R₄ are both CONQ₁Q₂,
or R₃ is CONQ₁Q₂ and then R₁, R₂, R₄ and R₅ are H, Br or I.

7. Chelate of a paramagnetic metal cation of a compound of formula I, and the salts thereof, according to claim 1, in which
m is 1,
R' is 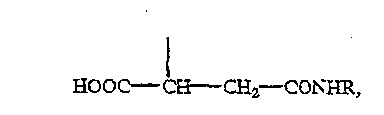 a is 1,
Z is selected from the group consisting of CH₂ and CH₂CONH,
Z' is CONH,
Z" is selected from the group consisting of CONH and CONH-CH₂-CONH,
R₁, R₃ and R₅ are Br,
R₂ and R₄ are CONQ₁Q₂ where Q₁ and Q₂ are hydroxylated C₂-C₆ alkyl groups together containing from 6 to 10 OH groups.

8. Chelate according to claim 1, in which the metal cation is Gd³⁺.

9. Gadolinium chelate, and the salts thereof, of the compound of formula I, according to claim 1, in which
m is 2
R' = 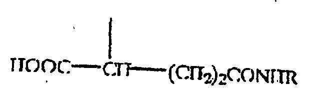 and R is

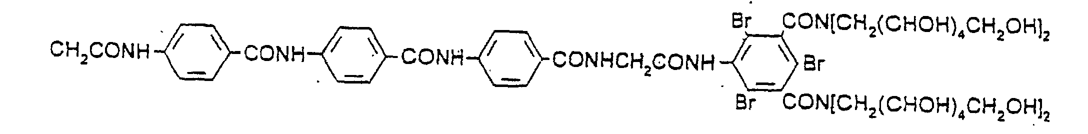

10. Gadolinium chelate, and the salts thereof, of the compound of formula I according to claim 1, in which
m is 2
R' is 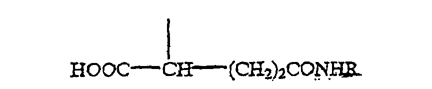 R = 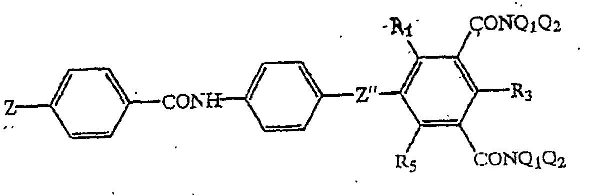 where Z" is CONH-CH₂CONH and Q₁ and Q₂ are CH₂(CHOH)₄CH₂OH, in which case either Z is CH₂ and R₁, R₃ and R₅ are I, or Z is CH₂CONH and R₁, R₃ and R₅ are Br, or where Z" is CONH, Z is selected from the group consisting of CH₂ and CH₂CONH and R₁, R₃ and R₅ are Br and Q₁ is CH₂(CHOH)₄CH₂OH and Q₂ is CH₂CHOH-CH₂OH or CH₂(CHOH)₄CH₂OH.

11. Contrast agent for diagnostic magnetic resonance imaging, comprising an effective amount of a chelate according to any of claims 1 to 10 in a pharmaceutically acceptable vehicle.

12. Scintigraphy agent containing a radio-labelled chelate of a metal cation with a compound of formula I as defined in claim 1

## Patentansprüche

1. Chelat eines Kations eines paramagnetischen Metalls mit einer Verbindung der Formel 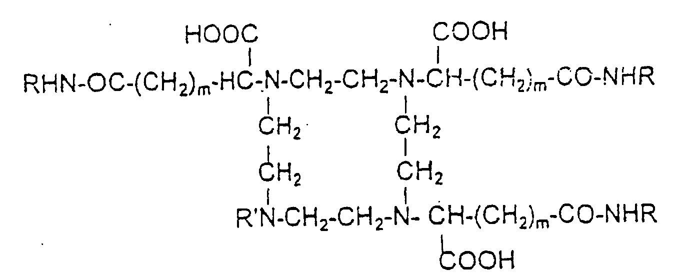 in der
m 1 oder 2 ist,
R'H, eine (C₁-C₄) Alkyl oder Hydroxyalkylgruppe, eine CH₂-COOH Gruppe oder CH₂-CONZ₁Z₂ ist, wobei Z₁ und Z₂ unabhängig voneinander H oder eine (C₁-C₄) Alkylgruppe, die gegebenenfalls hydroxyliert ist, sind
oder R' eine HOOC-CH-(CH₂)m-CONHR Gruppe ist,
R eine 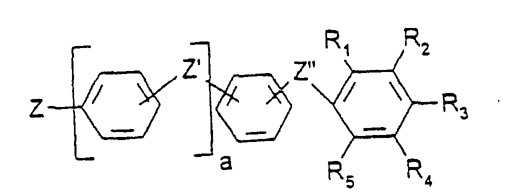 Gruppe ist,
in der a 1 oder 2 ist,
Z eine Bindung, CH₂, CH₂CONH oder (CH₂)₂NHCO ist,
Z' eine Bindung, O, S, NQ, CH₂, CO, CO-NQ, NQ-CO, NQ-CO-NQ oder CO-NQ-CH₂-CONQ ist,
Z'' CO-NQ, NQ-CO oder CO-NQ-CH₂-CO-NQ ist, wobei NQ-CO-CH₂-NQ-CO mit Q H oder einer (C₁-C₄) Alkylgruppe, die gegebenenfalls hydroxyliert ist, ist
R₁, R₂, R₃, R₄, R₅ jeweils unabhängig voneinander aus H, Br, Cl, I, CO-NQ₁Q₂ oder N(Q₁)-CO-Q₂ ausgewählt werden und Q₁ und
Q₂, die identisch oder unterschiedlich sind, aus den (C₂-C₆) Alkylgruppen, die gegebenenfalls hydroxyliert sind, die gegebenenfalls durch ein Sauerstoffatom unterbrochen sind ausgewählt werden, derart, dass Q₁ und Q₂ beide zusammen 4 bis 10 OH Gruppen umfassen, wobei verlangt wird, dass mindestens 1 und höchstens 2 Gruppen R₁, R₂, R₃, R₄, R₅ Amidgruppen sind, und ihren pharmazeutisch annehmbaren Salzen mit einer mineralischen Base oder einer organischen Base.

2. Chelat eines Kations eines paramagnetischen Metalls mit einer Verbindung der Formel I und ihren Salzen gemäß Anspruch 1, in der
m 2 ist,
R' 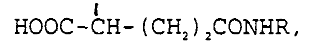 CH₂COOH oder CH₂CONZ₁Z₂ ist,
a 1 ist,
Z eine Bindung, CH₂ oder CH₂-CONH ist,
Z' CONH, NHCO, CONH-CH₂-CONH oder NHCONH ist,
Z'' CONH oder CONH-CH₂-CONH ist, und dann R₂ = R₄ = CONQ₁Q₂ und
R₁, R₃, R₅ H, Br, Cl oder I sind oder R₃= CONO₁O₂ und R₁, R₂, R₄, R₅ H, Br, Cl oder I sind, oder
Z'' NHCO ist und dann R₂ = R₄ = N(Q₁)COQ₂ und R₃ = N(Q₁)-COQ₂ und R₁, R₂, R₄, R₅ H, Br, Cl oder I sind.

3. Chelat eines paramagnetischen Metallkations mit einer Verbindung nach Formel I und ihren Salzen gemäß Anspruch 1, in der
m 2 ist,
R' 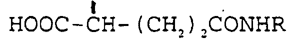 oder CH₂COOH ist,
a 1 ist,
Z eine Bindung, CH₂ oder CH₂CONH ist,
Z' CONH, NHCONH oder CONH-CH₂-CONH ist,
Z'' CONH oder CONH-CH₂-CONH ist,
R₁, R₃, R₅ jeweils alle drei Br oder I sind,
R₂ und R₄ CONQ₁Q₂ sind mit Q₁ und Q₂ ausgewählt aus den (C₂-C₆) hydroxylierten Alkylgruppen, die beide zusammen 6 bis 10 OH Gruppen umfassen oder 4 bis 8 OH Gruppen umfassen, wenn Q₁ und/oder Q₂ durch ein Sauerstoffatom unterbrochen sind.

4. Chelat eines paramagnetischen Metallkations mit einer Verbindung nach Formel I und ihren Salzen gemäß Anspruch 1, in der
m 2 ist
R' 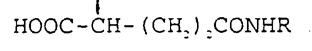 ist,
a 1 ist,
Z CH₂ oder CH₂CONH ist,
Z' CONH, NHCONH, ist,
Z'' CONH oder CONH-CH₂-CONH ist,
R₁, R₃ und R₅ alle drei Br oder I sind,
R₂ und R₄ CONQ₁Q₂ sind mit Q₁ und Q₂ ausgewählt aus den hydroxylierten (C₂-C₆) Alkylgruppen, die beide zusammen 6 bis 10 OH Gruppen umfassen oder 4 bis 8 OH Gruppen umfassen, wenn Q₁ und/oder Q₂ von einem Sauerstoffatom unterbrochen ist.

5. Chelat eines paramagnetischen Metallkations mit einer Verbindung nach Formel I und ihren Salzen gemäß Anspruch 1, in der
m 2 ist
R' CH₂CO₂H oder 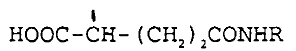 ist,
a 1 ist,
Z CH₂ oder CH₂CONH ist,
Z' und Z'' CONH sind,
R₁, R₃ und R₅ alle drei Br oder I sind,
R₂ und R₄ CONQ₁Q₂ sind mit Q₁ und Q₂ ausgewählt aus den hydroxylierten (C₂-C₆) Alkylgruppen, die beide zusammen 6 bis 10 OH Gruppen umfassen oder 4 bis 8 OH Gruppen umfassen, wenn Q₁ und/oder Q₂ durch ein Sauerstoffatom unterbrochen sind.

6. Chelat eines paramagnetischen Metallkations mit einer Verbindung nach Formel I und ihren Salzen gemäß Anspruch 1, in der
m 2 ist,
R' CH₂CO₂H oder 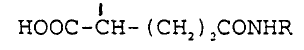 ist,
a 2 ist,
Z CH₂ oder CH₂CONH ist,
Z' CONH oder NHCO ist,
Z' ' CONH oder CONH-CH₂-CONH ist,
und R₁, R₃ und R₅ alle drei Br oder I sind,
und dann R₂ und R₄ beide CONQ₁Q₂ sind, oder
R₃ CONQ₁Q₂ ist, und dann R₁, R₂, R₄ und R₅ H, Br oder I sind.

7. Chelat eines paramagnetischen Metallkations mit einer Verbindung nach Formel I und ihren Salzen gemäß Anspruch 1, in der
m 1 ist,
R' 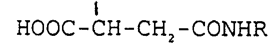 ist,
a 1 ist,
Z CH₂ oder CH₂CONH ist,
Z' CONH ist,
Z'' CONH oder CONH-CH₂-CONH ist,
R₁, R₃ und R₅ Br sind,
R₂ und R₄ CONQ₁Q₂ sind, wobei Q₁ und Q₂ hydroxylierte (C₂-C₆) Alkylgruppen sind, die beide zusammen 6 bis 10 OH Gruppen umfassen.

8. Chelat gemäß einem der Ansprüche 1 bis 7, in dem das Metallkation Gd³⁺ ist.

9. Gadolinium-Chelat und seine Salze der Verbindung gemäß Formel I gemäß Anspruch 1, in der
m 2 ist,
R' gleich HOOC-CH-(CH₂)₂CONHR,
und R 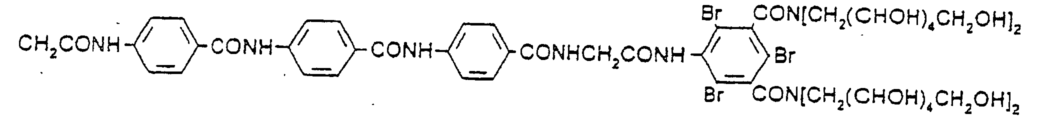 ist.

10. Gadolinium-Chelat und seine Salze der Verbindung gemäß Formel I gemäß Anspruch 1, in der
m 2 ist,
R' 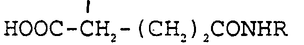 ist,
R = 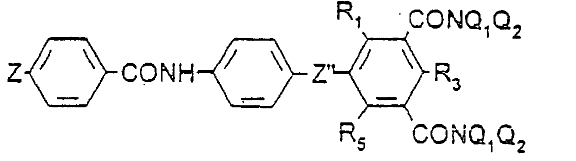
- wobei Z'' CONH-CH₂CONH ist, und Q₁ und Q₂ CH₂(CHOH)₄CH₂OH sind, dann entweder Z CH₂ ist und R₁, R₃ und R₅ I sind oder Z CH₂CONH ist, und R₁, R₃ und R₅ Br sind,
- oder wobei Z' ' CONH ist, Z CH₂ oder CH₂CONH ist und R₁, R₃, R₅ Br sind und Q₁ CH₂(CHOH)₄CH₂OH ist und Q₂ CH₂CHOH-CH₂OH oder CH₂ (CHOH)₄CH₂OH ist.

11. Kontrastmittel für die Bildgebungsdiagnostik für die magnetische Resonanz, das eine wirksame Menge eines Chelats nach einem der Ansprüche 1 bis 10 in einen pharmazeutisch annehmbaren Träger umfasst.

12. Szintigraphiemittel, das ein radiomarkiertes Chelat eines Metallkations mit einer Verbindung nach Formel I umfasst, wie sie in Anspruch 1 definiert ist.
